# EUROPEAN PATENT SPECIFICATION

(11) **EP 1 320 626 B1**
(45) Date of publication and mention of the grant of the patent: **04.11.2009**
(21) Application number: 01974228.7
(22) Date of filing: 05.09.2001
(51) Int. Cl.: C12P 13/04, C12P 13/08, C12N 1/21, C12N 15/11, C12N 15/60

(54) **FERMENTATION PROCESS FOR THE PREPARATION OF L-AMINO ACIDS USING STRAINS OF THE FAMILY ENTEROBACTERIACEAE**
FERMENTATIONSVERFAHREN ZUR HERSTELLUNG VON L-AMINOSÄUREN UNTER VERWENDUNG VON STÄMMEN AUS DER FAMILIE DER ENTEROBACTERIACEAE
PROCEDE DE FERMENTATION POUR PREPARER DES ACIDES AMINES L AU MOYEN DE SOUCHES DE LA FAMILLE DES ENTEROBACTERIACEAE

(30) Priority: 30.09.2000 DE 10048605; 09.11.2000 DE 10055516; 22.06.2001 DE 10130192
(43) Date of publication of application: 25.06.2003
(73) Proprietor: Evonik Degussa GmbH, 45128 Essen (DE)
(72) Inventor: RIEPING, Mechthild, 33619 Bielefeld (DE); BASTUCK, Christine, 33602 Bielefeld (DE); HERMANN, Thomas, 33739 Bielefeld (DE); THIERBACH, Georg, 33613 Bielefeld (DE)
(86) International application number: PCT/EP2001/010209
(87) International publication number: WO 2002/029080

(56) References cited:
- EP-A- 1 094 111
- DATABASE BIOSIS [Online] BIOSCIENCES INFORMATION SERVICE, PHILADELPHIA, PA, US; 1990 GOLDIE H ET AL: "PHYSICAL AND GENETIC ANALYSIS OF THE PHOSPHOENOLPYRUVATE CARBOXYKINASE PCK-A LOCUS FROM ESCHERICHIA-COLI K12" Database accession no. PREV199089092769 XP002193202 & MOLECULAR & GENERAL GENETICS, vol. 220, no. 2, 1990, pages 191-196, ISSN: 0026-8925
- KRAMER R: "Genetic and physiological approaches for the production of amino acids" JOURNAL OF BIOTECHNOLOGY, ELSEVIER SCIENCE PUBLISHERS, AMSTERDAM, NL, vol. 45, no. 1, 12 February 1996 (1996-02-12), pages 1-21, XP004036833 ISSN: 0168-1656
- DATABASE BIOSIS [Online] BIOSCIENCES INFORMATION SERVICE, PHILADELPHIA, PA, US; 1990 MEDINA V ET AL: "SEQUENCE OF THE PCK-A GENE OF ESCHERICHIA-COLI K-12 RELEVANCE TO GENETIC AND ALLOSTERIC REGULATION AND HOMOLOGY OF ESCHERICHIA-COLI PHOSPHOENOLPYRUVATE CARBOXYKINASE WITH THE ENZYMES FROM TRYPANOSOMA-BRUCEI AND SACCHAROMYCES-CEREVISIAE" Database accession no. PREV199191038347 XP002193203 cited in the application & JOURNAL OF BACTERIOLOGY, vol. 172, no. 12, 1990, pages 7151-7156, ISSN: 0021-9193
- DATABASE BIOSIS [Online] BIOSCIENCES INFORMATION SERVICE, PHILADELPHIA, PA, US; March 1999 (1999-03) PROST JEAN-FRANCOIS ET AL: "Detection of an extended -10 element in the promoter region of the pckA gene encoding phosphoenolpyruvate carboxykinase in Escherichia coli." Database accession no. PREV199900300021 XP002193204 & BIOCHIMIE (PARIS), vol. 81, no. 3, March 1999 (1999-03), pages 197-200, ISSN: 0300-9084
- DATABASE CA [Online] CHEMICAL ABSTRACTS SERVICE, COLUMBUS, OHIO, US; RIVOLTA, CARLO ET AL: "A 35.7 kb DNA fragment from the Bacillus subtilis chromosome containing a putative 12.3 kb operon involved in hexuronate catabolism and a perfectly symmetrical hypothetical catabolite-responsive element" retrieved from STN Database accession no. 129:63856 XP002197824 & MICROBIOLOGY (READING, U. K.) (1998), 144(4), 877-884 ,
- DATABASE BIOSIS [Online] BIOSCIENCES INFORMATION SERVICE, PHILADELPHIA, PA, US; 1991 BLOMFIELD I C ET AL: "ALLELIC EXCHANGE IN ESCHERICHIA-COLI USING THE BACILLUS-SUBTILIS SAC-B GENE AND A TEMPERATURE-SENSITIVE PSC-101 REPLICON" Database accession no. PREV199192075325 XP002197825 & MOLECULAR MICROBIOLOGY, vol. 5, no. 6, 1991, pages 1447-1458, ISSN: 0950-382X

## Description

### Field of the Invention

The present invention relates to a fermentation process for the preparation of L-amino acids, especially L-threonine, using strains of the family Enterobacteriaceae in which at least the pckA gene is attenuated.

### Prior Art

L-Amino acids are used in animal nutrition, in human medicine and in the pharmaceutical industry.

It is known to prepare L-amino acids by the fermentation of strains of Enterobacteriaceae, especially Escherichia coli and Serratia marcescens. Because of their great importance, attempts are constantly being made to improve the preparative processes. Improvements to the processes may relate to measures involving the fermentation technology, e.g. stirring and oxygen supply, or the composition of the nutrient media, e.g. the sugar concentration during fermentation, or the work-up to the product form, e.g. by ion exchange chromatography, or the intrinsic productivity characteristics of the microorganism itself.

The productivity characteristics of these microorganisms are improved by using methods of mutagenesis, selection and mutant choice to give strains which are resistant to antimetabolites, e.g. the threonine analogue α-amino-β-hydroxyvaleric acid (AHV) or auxotrophic for metabolites of regulatory significance, and produce L-amino acids, e.g. L-threonine.

Methods of recombinant DNA technology have also been used for some years to improve L-amino acid-producing strains of the family Enterobacteriaceae by amplifying individual amino acid biosynthesis genes and studying the effect on production.

EP-A-1 094 111 provides a process for the fermentative preparation of L-amino acids, in particular L-lysine and L-threonine, using coryneform bacteria which in particular already produce the L-amino acids and in which the nucleotide sequence(s) which code(s) for the pck gene product are attenuated.

Prost Jean-Francois et al: "Detection of an extended - 10 element in the promoter region of the pckA gene encoding phosphoenolpyruvate carboxykinase in Escherichia Coli." Biochemie 81: 197-200 (1999), describe that the regulation of transcription of the pckA gene coding for phosphoenolpyruvate carboxykinase in Escherchia coli was analysed by site-directed mutagenesis of the promoter region and measurement of in vitro transcription initiation.

Kramer R: "Genetic and physiological approaches for the production of amino acids" Journal of Biotechnology, Eslevier Science Publishers, Amsterdam, NL, vol. 45, no. 1, 12 February (1996-02-12), pages 1-21, XP004036833 ISSN: 0168-1656, describes the relevant pathways in biotechnologically relevant amino acid-producing microorganisms.

Database Biosis [Online] Biosciences Information Service, Philadelphia, PA, US; 1990, Medina V. et al. provides "Sequence of the pck - a gene of Escherichia-Coli K-12 Relevance to genetic and allosteric regulation and Homology of Escherichia Coli Phosphoenolpyruvate Carboxykinase with the enzymes from Trypanosoma-Brucei and Saccharomyces-Cerevisiae" XP002193203 Database accession no. PREV199191038347.

In Database Biosis [Online] Biosciences Information Service, Philadelphia, PA, US; 1990, Goldie H. et al: "Physical and genetic locus from Escherichia-Coli K12" XP002193202 Database accession no. PREV199089092769 can be found.

### Object of the Invention

The object which the inventors set themselves was to provide novel procedures for improving the preparation of L-amino acids, especially L-threonine, by fermentation.

### Summary of the Invention

The invention provides a fermentation process for the preparation of L-amino acids, especially L-threonine, using microorganisms of the family Enterobacteriaceae which, in particular, already produce L-threonine and in which the nucleotide sequence (pckA gene) coding for the enzyme phosphoenolpyruvate carboxykinase (PEP carboxykinase) (EC 4.1.1.49) is attenuated.

### Detailed Description of the Invention

Where L-amino acids or amino acids are mentioned in the following, this means one or more amino acids, including their salts, chosen from the group consisting of L-asparagine, L-threonine, L-serine, L-glutamate, L-glycine, L-alanine, L-cysteine, L-valine, L-methionine, L-isoleucine, L-leucine, L-tyrosine, L-phenylalanine, L-histidine, L-lysine, L-tryptophan, L-homoserine and L-arginine. L-Threonine is particularly preferred.

In this context the term "attenuation" describes the reduction or switching-off, in a microorganism, of the intracellular activity of one or more enzymes (proteins) which are coded for by the appropriate DNA, for example by using a weak promoter or a gene or allele which codes for an appropriate enzyme with low activity, or inactivating the appropriate enzyme (protein), and optionally combining these measures.

By attenuation measures, the activity or concentration of the corresponding protein is in general reduced to 0 to 75%, 0 to 50%, 0 to 25%, 0 to 10% or 0 to 5% of the activity or concentration of the wild-type protein or of the activity or concentration of the protein in the starting microorganism.

The process is characterized in that the following steps are carried out:
a) fermentation of the microorganisms of the family Enterobacteriaceae producing the desired L-amino acid, in which microorganisms at least the pckA gene or nucleotide sequences coding for the pckA gene product are attenuated.
b) enrichment of the L-amino acid in the medium or in the bacterial cells, and
c) isolation of the L-amino acid or
d) constituents of the fermentation broth and the biomass in its entirety or portions thereof being isolated as a solid product together with the L-amino acid.

The microorganisms provided by the present invention can produce L-amino acids from glucose, sucrose, lactose, fructose, maltose, molasses, optionally starch or optionally cellulose, or from glycerol and ethanol. Said microorganisms are representatives of the family Enterobacteriaceae selected from the genera Escherichia, Erwinia, Providencia and Serratia. The genera Escherichia and Serratia are preferred. The species Escherichia coli and Serratia marcescens may be mentioned in particular among the genera Escherichia and Serratia respectively.

Examples of suitable strains, particularly L-threonine-producing strains, of the genus Escherichia, especially of the species Escherichia coli, are:
Escherichia coli TF427
Escherichia coli H4578
Escherichia coli KY10935
Escherichia coli VNIIgenetika MG442
Escherichia coli VNIIgenetika M1
Escherichia coli VNIIgenetika 472T23
Escherichia coli BKIIM B-3996
Escherichia coli kat 13
Escherichia coli KCCM-10132.

Examples of suitable L-threonine-producing strains of the genus Serratia, especially of the species Serratia marcescens, are:
Serratia marcescens HNr21
Serratia marcescens TLr156
Serratia marcescens T2000.

L-Threonine-producing strains of the family Enterobacteriaceae preferably possess, inter alia, one or more genetic or phenotypic characteristics selected from the group comprising resistance to α-amino-β-hydroxyvaleric acid, resistance to thialysine, resistance to ethionine, resistance to α-methylserine, resistance to diaminosuccinic acid, resistance to α-aminobutyric acid, resistance to borrelidine, resistance to rifampicin, resistance to valine analogues such as valine hydroxamate, resistance to purine analogues such as 6-dimethylaminopurine, need for L-methionine, optionally partial and compensable need for L-isoleucine, need for meso-diaminopimelic acid, auxotrophy in respect of threonine-containing dipeptides, resistance to L-threonine, resistance to L-homoserine, resistance to L-lysine, resistance to L-methionine, resistance to L-glutamic acid, resistance to L-aspartate, resistance to L-leucine, resistance to L-phenylalanine, resistance to L-serine, resistance to L-cysteine, resistance to L-valine, sensitivity to fluoropyruvate, defective threonine dehydrogenase, optionally capability for sucrose utilization, amplification of the threonine operon, amplification of homoserine dehydrogenase I-aspartate kinase I, preferably of the feedback-resistant form, amplification of homoserine kinase, amplification of threonine synthase, amplification of aspartate kinase, optionally of the feedback-resistant form, amplification of aspartate semialdehyde dehydrogenase, amplification of phosphoenolpyruvate carboxylase, optionally of the feedback-resistant form, amplification of phosphoenolpyruvate synthase, amplification of transhydrogenase, amplification of the RhtB gene product, amplification of the RhtC gene product, amplification of the YfiK gene product, amplification of malate quinone oxidoreductase and amplification of a pyruvate carboxylase and attenuation of acetic acid formation.

It has been found that the production of L-amino acids, especially L-threonine, by microorganisms of the family Enterobacteriaceae is improved after attenuation and, in particular, switching-off of the pckA gene coding for PEP carboxykinase (EC 4.1.1.49).

The nucleotide sequence of the pckA gene of Escherichia coli has been published by Medina et al. (Journal of Bacteriology 172, 7151-7156 (1990)) and can also be taken from the genome sequence of Escherichia coli published by Blattner et al. (Science 277, 1453-1462 (1997)). The nucleotide sequence of the pckA gene of Escherichia coli is represented in SEQ ID No. 1 and the amino acid sequence of the corresponding gene product is represented in SEQ ID No. 2.

The pckA genes described in the above literature references can be used according to the invention. It is also possible to use alleles of the pckA gene which result from the degeneracy of the genetic code or from neutral sense mutations.

Attenuation can be achieved for example by reducing or switching off the expression of the pckA gene or the catalytic properties of the enzyme protein. Both measures may optionally be combined.

Gene expression can be reduced by an appropriate culture technique, by genetic modification (mutation) of the signal structures of gene expression, or by means of antisense RNA technology. Examples of signal structures of gene expression are repressor genes, activator genes, operators, promoters, attenuators, ribosome binding sites, the start codon and terminators. Those skilled in the art will find relevant information inter alia in e.g. Jensen and Hammer (Biotechnology and Bioengineering 58, 191-195 (1998)), Carrier and Keasling (Biotechnology Progress 15, 58-64 (1999)), Franch and Gerdes (Current Opinion in Microbiology 3, 159-164 (2000)) and well-known textbooks on genetics and molecular biology, for example the textbook by Knippers ("Molekulare Genetik" ("Molecular Genetics"), 6th edition, Georg Thieme Verlag, Stuttgart, Germany, 1995) or the textbook by Winnacker ("Gene und Klone" ("From Genes to Clones"), VCH Verlagsgesellschaft, Weinheim, Germany, 1990).

Mutations which cause a change or reduction in the catalytic properties of enzyme proteins are known from the state of the art. Examples which may be mentioned are the studies of Qiu and Goodman (Journal of Biological Chemistry 272, 8611-8617 (1997)), Yano et al. (Proceedings of the National Academy of Sciences USA 95, 5511-5515 (1998)) and Wente and Schachmann (Journal of Biological Chemistry 266, 20833-20839 (1991)). Surveys can be found in well-know textbooks on genetics and molecular biology, e.g. the textbook by Hagemann ("Allgemeine Genetik" ("General Genetics"), Gustav Fischer Verlag, Stuttgart, 1986).

Mutations to be taken into consideration are transitions, transversions, insertions and deletions. Depending on the effect of amino acid exchange on the enzyme activity, the term missense mutations or nonsense mutations is used.
Insertions or deletions of at least one base pair in a gene cause frame shift mutations, the result of which is that false amino acids are incorporated or translation is terminated prematurely. Deletions of several codons typically lead to a complete loss of enzyme activity.
Instructions for the production of such mutations form part of the state of the art and can be found in well-known textbooks on genetics and molecular biology, e.g. the textbook by Knippers ("Molekulare Genetik" ("Molecular Genetics"), 6th edition, Georg Thieme Verlag, Stuttgart, Germany, 1995), the textbook by Winnacker ("Gene und Klone" ("From Genes to Clones"), VCH Verlagsgesellschaft, Weinheim, Germany, 1990) or the textbook by Hagemann ("Allgemeine Genetik" ("General Genetics"), Gustav Fischer Verlag, Stuttgart, 1986).

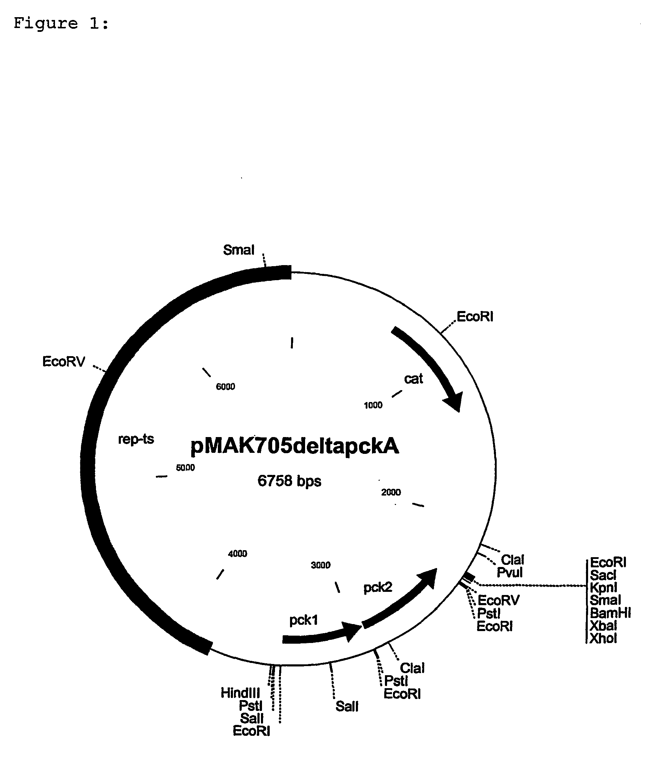

An example of a plasmid by means of which the pckA gene of Escherichia coli can be attenuated and, in particular, switched off by position-specific mutagenesis is plasmid pMAK705ApckA (Figure 1). It contains only part of the 5' region and part of the 3' region of the pckA gene. A 349 bp segment of the coding region is missing (deletion).
The sequence of this DNA, which can be used for mutagenesis of the pckA gene, is represented in SEQ ID No. 3.

The deletion mutation of the pckA gene can be incorporated into suitable strains by gene or allele exchange.

A common method is the method of gene exchange using a conditionally replicating pSC101 derivative, pMAK705, as described by Hamilton et al. (Journal of Bacteriology 174, 4617-4622 (1989)). Other methods described in the state of the art, for example that of Martinez-Morales et al. (Journal of Bacteriology, 7143-7148 (1999)) or that of Boyd et al. (Journal of Bacteriology 182, 842-847 (2000)), can also be used.

When exchange has been carried out, the form of the ΔPckA allele represented in SEQ ID No. 4, which is a further subject of the invention, is present in the strain in question.

Mutations in the pckA gene or mutations involving expression of the pckA gene can also be transferred to different strains by conjugation or transduction.

Furthermore, for the production of L-amino acids, especially L-threonine, with strains of the family Enterobacteriaceae, it can be advantageous not only to attenuate the pckA gene but also to amplify one or more enzymes of the known threonine biosynthetic pathway, or enzymes of the anaplerotic metabolism, or enzymes for the production of reduced nicotinamide adenine dinucleotide phosphate.

In this context the term "amplification" describes the increase in the intracellular activity, in a microorganism, of one or more enzymes or proteins which are coded for by the appropriate DNA, for example by increasing the copy number of the gene(s), using a strong promoter or using a gene coding for an appropriate enzyme or protein with a high activity, and optionally combining these measures.

By amplification measures, in particular over-expression, the activity or concentration of the corresponding protein is in general increased by at least 10%, 25%, 50%, 75%, 100%, 150%, 200%, 300%, 400% or 500%, up to a maximum of 1000% or 2000%, based on that of the wild-type protein or the activity or concentration of the protein in the starting microorganism.

Thus, for example, one or more genes selected from the group comprising:
- the thrABC operon coding for aspartate kinase, homoserine dehydrogenase, homoserine kinase and threonine synthase (US-A-4,278,765),
- the pyc gene coding for pyruvate carboxylase DE-A-19 831 609),
- the pps gene coding for phosphoenolpyruvate synthase (Molecular and General Genetics 231, 332 (1992)),
- the ppc gene coding for phosphoenolpyruvate carboxylase (Gene 31, 279-283 (1984)),
- the pntA and pntB genes coding for transhydrogenase (European Journal of Biochemistry 158, 647-653 (1986));
- the rhtB gene for homoserine resistance (EP-A-0994190), and
- the rhtC gene for threonine resistance (EP-A-1013765),
- the gdhA gene coding for glutamate dehydrogenase (Gene 27:193-199 (1984)
   can be simultaneously amplified and, in particular, overexpressed.

Furthermore, for the production of L-amino acids, especially L-threonine, it can be advantageous not only to attenuate the pckA gene but also to attenuate and, in particular, switch off one or more genes selected from the group comprising:
- the tdh gene coding for threonine dehydrogenase (Ravnikar and Somerville, Journal of Bacteriology 169, 4716-4721 (1987)),
- the mdh gene coding for malate dehydrogenase (EC 1.1.1.37) (Vogel et al., Archives in Microbiology 149, 36-42 (1987)),
- the gene product of the open reading frame (orf) yjfA (Accession Number AAC77180 of the National Center for Biotechnology Information (NCBI, Bethesda, MD, USA) and SEQ ID No. 5), and
- the gene product of the open reading frame (orf) ytfP (Accession Number AAC77179 of the National Center for Biotechnology Information (NCBI, Bethesda, MD, USA) and SEQ ID No. 5),
   or to reduce the expression.

It is preferred to attenuate the open reading frame yjfA and/or the open reading frame ytfP.

It is also possible according to the description to attenuate the open reading frames yjfA and/or ytfP independently of the pckA gene, in order to achieve an improvement in the amino acids, in particular L-threonine production.

The description accordingly also provides a process, characterized in that the following steps are carried out:
d) fermentation of microorganisms of the Enterobacteriaceae family in which at least the open reading frame yjfA and/or ytfP is attenuated,
e) enrichment of the L-amino acid in the medium or in the cells of the microorganisms of the Enterobacteriaceae family, and
f) isolation of the L-threonine, constituents of the fermentation broth and the biomass in its entirety or portions thereof optionally being isolated as a solid product together with the L-amino acid.

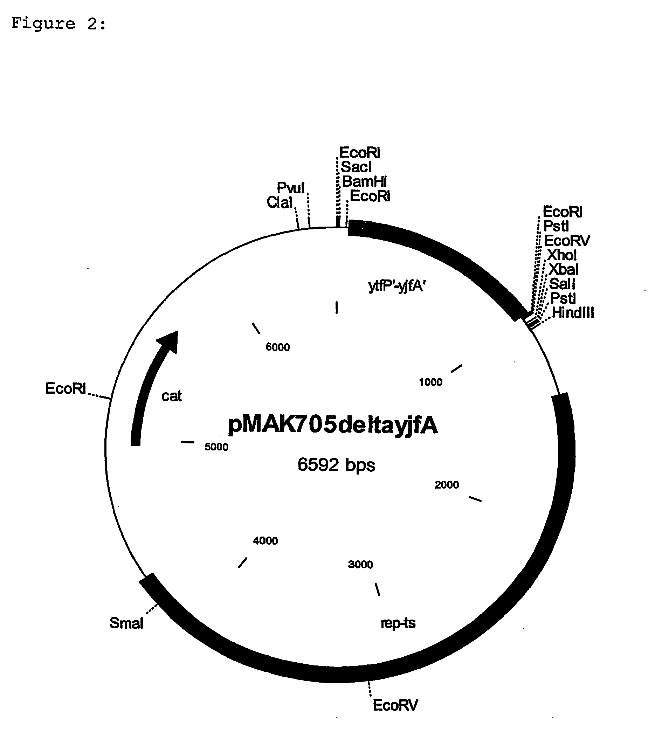

An example of a plasmid by means of which the open reading frames yjfA and ytfP of Escherichia coli can be attenuated and, in particular, switched off by position-specific mutagenesis is plasmid pMAK705ΔyjfA (Figure 2). It contains only the 5' and 3' flanks of the ytfP-yjfA region, including very short residues of the open reading frames yjfA and ytfP. A 337 bp long part of the ytfP-yjfA region is missing (deletion). The sequence of this DNA, which can be used for mutagenesis of the ytfP-yjfA region, is represented in SEQ ID No. 6.

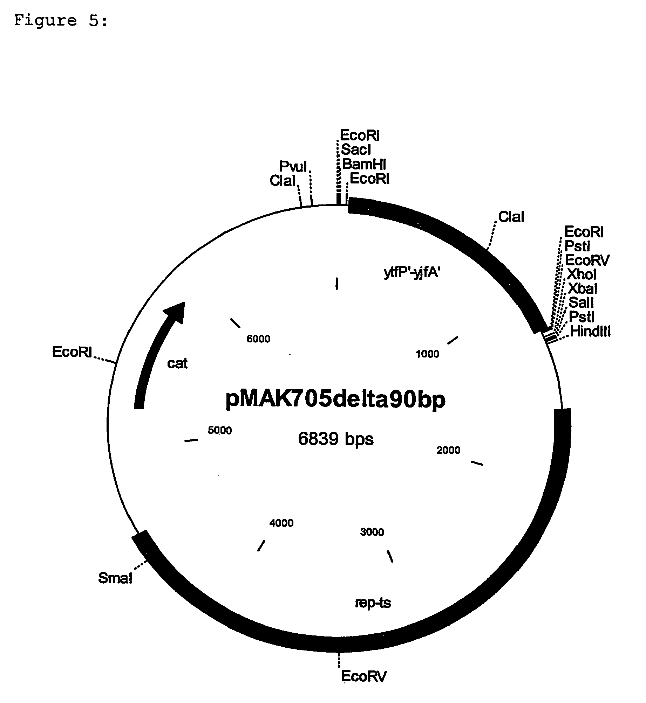

An further example of a plasmid by means of which the open reading frames yjfA and ytfP of Escherichia coli can be attenuated and, in particular, switched off by position-specific mutagenesis is the plasmid pMAK705Δ90bp (Figure 5). It also contains only the 5' and 3' flanks of the ytfP-yjfA region including very short residues of the open reading frames yjfA and ytfP. A 90 bp long part of the ytfP-yjfA region is missing (deletion). The sequence of this DNA, which can be used for mutagenesis of the ytfP-yjfA region, is represented in SEQ ID No. 7.

This deletion mutation can be incorporated into suitable strains by gene or allele replacement. It is also possible to transfer mutations in the open reading frames yjfA and/or ytfP or mutations affecting expression of these open reading frames into various strains by conjugation or transduction.

When replacement has been carried out, the form of the ΔytfP and ΔyjfA allele represented in SEQ ID No. 6 or SEQ ID No. 7, which are a further subject of the description is present in the strain in question.

Furthermore, for the production of L-amino acids, especially L-threonine, it can be advantageous, in addition to the individual or joint attenuation of the pckA gene or of the open reading frames yjfA and/or ytfP, to switch off undesired secondary reactions (Nakayama: "Breeding of Amino Acid Producing Microorganisms", in: Overproduction of Microbial Products, Krumphanzl, Sikyta, Vanek (eds.), Academic Press, London, UK, 1982).

The microorganisms prepared according to the invention can be cultivated by the batch process or the fed batch process. A summary of known cultivation methods is provided in the textbook by Chmiel (Bioprozesstechnik 1. Einführung in die Bioverfahrenstechnik (Bioprocess Technology 1. Introduction to Bioengineering) (Gustav Fischer Verlag, Stuttgart, 1991)) or in the textbook by Storhas (Bioreaktoren und periphere Einrichtungen (Bioreactors and Peripheral Equipment) (Vieweg Verlag, Brunswick/Wiesbaden, 1994)).

The culture medium to be used must appropriately meet the demands of the particular strains. Descriptions of culture media for various microorganisms can be found in the handbook "Manual of Methods for General Bacteriology" of the American Society for Bacteriology (Washington DC, USA, 1981).

Carbon sources which can be used are sugars and carbohydrates, e.g. glucose, sucrose, lactose, fructose, maltose, molasses, starch and optionally cellulose, oils and fats, e.g. soya oil, sunflower oil, groundnut oil and coconut fat, fatty acids, e.g. palmitic acid, stearic acid and linoleic acid, alcohols, e.g. glycerol and ethanol, and organic acids, e.g. acetic acid. These substances can be used individually or as a mixture.

Nitrogen sources which can be used are organic nitrogen-containing compounds such as peptones, yeast extract, meat extract, malt extract, corn steep liquor, soya bean flour and urea, or inorganic compounds such as ammonium sulfate, ammonium chloride, ammonium phosphate, ammonium carbonate and ammonium nitrate. The nitrogen sources can be used individually or as a mixture.

Phosphorus sources which can be used are phosphoric acid, potassium dihydrogenphosphate or dipotassium hydrogenphosphate or the corresponding sodium salts. The culture medium must also contain metal salts, e.g. magnesium sulfate or iron sulfate, which are necessary for growth. Finally, essential growth-promoting substances such as amino acids and vitamins can be used in addition to the substances mentioned above. Suitable precursors can also be added to the culture medium. Said feed materials can be added to the culture all at once or fed in appropriately during cultivation.

The pH of the culture is controlled by the appropriate use of basic compounds such as sodium hydroxide, potassium hydroxide, ammonia or aqueous ammonia, or acid compounds such as phosphoric acid or sulfuric acid. Foaming can be controlled using antifoams such as fatty acid polyglycol esters. The stability of plasmids can be maintained by adding suitable selectively acting substances, e.g. antibiotics, to the medium. Aerobic conditions are maintained by introducing oxygen or oxygen-containing gaseous mixtures, e.g. air, into the culture. The temperature of the culture is normally 25°C to 45°C and preferably 30°C to 40°C. The culture is continued until the formation of L-amino acids or L-threonine has reached a maximum. This objective is normally achieved within 10 hours to 160 hours.

L-Amino acids can be analyzed by means of anion exchange chromatography followed by ninhydrin derivation, ass described by Spackman et al. (Analytical Chemistry 30, 1190 (1958)), or by reversed phase HPLC, as described by Lindroth et al. (Analytical Chemistry 51, 1167-1174 (1979)).

A pure culture of the Escherichia coli K-12 strain DHSα/pMAK705 was deposited on 8th September 2000 at the Deutsche Sammlung für Mikroorganismen und Zellkulturen (DSMZ = German Collection of Microorganisms and Cell Cultures, Braunschweig, Germany) in accordance with the Budapest Treaty as DSM 13720.

A pure culture of the Escherichia coli K-12 strain MG442ΔpckA was deposited on 2nd October 2000 at the Deutsche Sammlung für Mikroorganismen und Zellkulturen. (DSMZ = German Collection of Microorganisms and Cell Cultures, Braunschweig, Germany) in accordance with the Budapest Treaty as DSM 13761.

A pure culture of the Escherichia coli K-12 strain B-3996kurΔtdhΔpckA/pVIC40 was deposited on 9th March 2001 at the Deutsche Sammlung für Mikroorganismen und Zellkulturen (DSMZ = German Collection of Microorganisms and Cell Cultures, Braunschweig, Germany) in accordance with the Budapest Treaty as DSM 14150.

A pure culture of the Escherichia coli K-12 strain MG442Δ90yjfA was deposited on 9th May 2001 at the Deutsche Sammlung für Mikroorganismen und Zellkulturen (DSMZ = German Collection of Microorganisms and Cell Cultures, Braunschweig, Germany) in accordance with the Budapest Treaty as DSM 14289.

It is also possible according to the description individually to attenuate the open reading frames ytfP and yjfA in order to improve the production of L-amino acids.

The process according to the invention is used for the preparation of L-amino acids, e.g. L-threonine, L-isoleucine, L-methionine, L-homoserine and L-lysine, especially L-threonine, by fermentation.

The present invention is illustrated in greater detail below with the aid of Examples.

The isolation of plasmid DNA from Escherichia coli and all the techniques for restriction, Klenow treatment and alkaline phosphatase treatment were carried out as described by Sambrook et al. (Molecular cloning - A laboratory manual (1989), Cold Spring Harbor Laboratory Press). Unless indicated otherwise, the transformation of Escherichia coli was carried out as described by Chung et al. (Proceedings of the National Academy of Sciences USA 86, 2172-2175 (1989)).

The incubation temperature for the preparation of strains and transformants was 37°C. Temperatures of 30°C and 44°C were used in the gene exchange process of Hamilton et al.

### Example 1

### Construction of the deletion mutation of the pckA gene

Parts of the 5' and 3' regions of the pckA gene of Escherichia coli K12 were amplified using the polymerase chain reaction (PCR) and synthetic oligonucleotides. The nucleotide sequence of the pckA gene in E. coli K12 MG1655 (SEQ ID No. 1) was used to synthesize the following PCR primers (MWG Biotech, Ebersberg, Germany):
pckA'5'-1: 5' - GATCCGAGCCTGACAGGTTA - 3'
pckA'5'-2: 5' - GCATGCGCTCGGTCAGGTTA - 3'
pckA'3'-1: 5' - AGGCCTGAAGATGGCACTATCG - 3'
pckA'3'-2: 5' - CCGGAGAAGCGTAGGTGTTA - 3'.

The chromosomal E. coli K12 MG1655 DNA used for the PCR was isolated with "Qiagen Genomic-tips 100/G" (QIAGEN, Hilden, Germany) according to the manufacturer's instructions. An approx. 500 bp DNA fragment from the 5' region of the pckA gene (denoted as pck1) and an approx. 600 bp DNA fragment from the 3' region of the pckA gene (denoted as pck2) could be amplified with the specific primers under standard PCR conditions (Innis et al. (1990), PCR Protocols. A Guide to Methods and Applications, Academic Press) using Taq DNA polymerase (Gibco-BRL, Eggenstein, Germany). The PCR products were each ligated with vector pCR2.1TOPO (TOPO TA Cloning Kit, Invitrogen, Groningen, The Netherlands) according to the manufacturer's instructions and transformed into E. coli strain TOP10F'. Plasmid-carrying cells were selected on LB agar containing 50 µg/ml of ampicillin. After isolation of the plasmid DNA, vector pCR2.1TOPOpck2 was cleaved with the restriction enzymes StuI and XbaI and, after separation in 0.8% agarose gel, the pck2 fragment was isolated with the aid of the QIAquick Gel Extraction Kit (QIAGEN, Hilden, Germany). After isolation of the plasmid DNA, vector pCR2.1TOPOpckl was cleaved with the enzymes EcoRV and XbaI and ligated to the isolated pck2 fragment. The E. coli strain DH5α was transformed with the ligation mixture and plasmid-carrying cells were selected on LB agar containing 50 µg/ml of ampicillin. After isolation of the plasmid DNA, control cleavage with the enzymes SpeI and XbaI was used to detect plasmids containing in cloned form, the mutagenic DNA sequence represented in SEQ ID No. 3. One of the plasmids was denoted as pCR2.lTOPOΔpckA.

### Example 2

### Construction of exchange vector pMAK705ΔpckA

After restriction with the enzymes SpeI and XbaI and separation in 0.8% agarose gel, the pckA allele described in Example 1 was isolated from vector pCR2.1TOPOΔpckA and ligated to plasmid pMAK705 (Hamilton et al., Journal of Bacteriology 174, 4617-4622 (1989)) which had been digested with the enzyme XbaI. DH5α was transformed with the ligation mixture and plasmid-carrying cells were selected on LB agar containing 20 µg/ml of chloramphenicol. After isolation of the plasmid DNA and cleavage with the enzymes HpaI, KpnI, HindIII, SalI and PstI, successful cloning was detected. The exchange vector formed, (= pMAK705deltapckA), is shown in Figure 1.

### Example 3

Position-specific mutagenesis of the pckA gene in the E. coli strain MG442

The L-threonine-producing E. coli strain MG442 is described in patent US-A-4,278,765 and deposited in the Russian National Collection of Industrial Microorganisms (VKPM, Moscow, Russia) as CMIM B-1628.

The strain MG442 has a resistance to α-amino-β-hydroxyvaleric acid and has an optionally partial and compensable need for L-isoleucine.

For exchange of the chromosomal pckA gene for the plasmid-coded deletion construct, MG442 was transformed with plasmid pMAK705ΔpckA. The gene exchange was carried out by the selection method described by Hamilton et al. (Journal of Bacteriology 174, 4617-4622 (1989)) and was verified by standard PCR methods (Innis et al. (1990), PCR Protocols. A Guide to Methods and Applications, Academic Press) using the following oligonucleotide primers:
pckA'5'-1: 5' - GATCCGAGCCTGACAGGTTA - 3'
pckA'3'-2: 5' - CCGGAGAAGCGTAGGTGTTA - 3'

The strain obtained was denoted as MG442ΔpckA.

### Example 4

### Preparation of L-threonine with the strain MG442ΔpckA

MG442ΔpckA was cultivated on minimum medium of the following composition: 3.5 g/l of "Na₂HPO₄·2H₂O, 1.5 g/l of KH₂PO₄, 1 g/l of NH₄Cl, 0.1 g/l of MgSO₄·7H₂O, 2 g/l of glucose and 20 g/l of agar. The formation of L-threonine was checked in 10 ml batch cultures contained in 100 ml Erlenmeyer flasks. These were inoculated with 10 ml of a preculture medium of the following composition: 2 g/l of yeast extract, 10 g/l of (NH₄)₂SO₄, 1 g/l of KH₂PO₄, 0.5 g/l of MgSO₄·7H₂O, 15 g/l of CaCO₃ and 20 g/l of glucose, and incubated for 16 hours at 37°C and 180 rpm on an ESR incubator from Kühner AG (Birsfelden, Switzerland). 250 µl of this preculture were transferred to 10 ml of a production medium (25 g/l of (NH₄)₂SO₄, 2 g/l of KH₂PO₄, 1 g/l of MgSO₄·7H₂O, 0.03 g/l of FeSO₄·7H₂O, 0.018 g/l of MnSO₄·1H₂O, 30 g/l of CaCO₃, 20 g/l of glucose) and incubated for 48 hours at 37°C. After incubation, the optical density (OD) of the culture suspension was determined with an LP2W photometer from Dr. Lange (Berlin, Germany) at a measurement wavelength of 660 nm.

The concentration of L-threonine formed was then determined in the sterile-filtered culture supernatant with an amino acid analyzer from Eppendorf-BioTronik (Hamburg, Germany) by means of ion exchange chromatography and postcolumn reaction with ninhydrin detection.

The result of the experiment is shown in Table 1.

**Table 1**

| Strain | OD (660 nm) | L-Threonine g/l |
|---|---|---|
| MG442 | 6.0 | 1.5 |
| MG442ΔpckA | 5.4 | 3.7 |

### Example 5

### Preparation of L-threonine with the strain MG442ΔpckA/pMW218gdhA

### 5.1 Amplification and cloning of the gdhA gene

The glutamate dehydrogenase gene from Escherichia coli K12 is amplified using the polymerase chain reaction (PCR) and synthetic oligonucleotides. Starting from the nucleotide sequence for the gdhA gene in E. coli K12 MG1655 (gene library: Accession No. AE000270 and No. AE000271) PCR primers are synthesized (MWG Biotech, Ebersberg, Germany):
Gdh1: 5' - TGAACACTTCTGGCGGTACG - 3'
Gdh2: 5' - CCTCGGCGAAGCTAATATGG - 3'

The chromosomal E. coli K12 MG1655 DNA employed for the PCR is isolated according to the manufacturers instructions with "QIAGEN Genomic-tips 100/G" (QIAGEN, Hilden, Germany). A DNA fragment approx. 2150 bp in size, which comprises the gdhA coding region and approx. 350 bp 5'-flanking and approx. 450 bp 3'-flanking sequences, can be amplified with the specific primers under standard PCR conditions (Innis et al.: PCR Protocols. A Guide to Methods and Applications, 1990, Academic Press) with the Pfu-DNA polymerase (Promega Corporation, Madison, USA). The PCR product is cloned in the plasmid pCR2.1TOPO and transformed in the E. coli strain TOP10 (Invitrogen, Leek, The Netherlands, Product Description TOPO TA Cloning Kit, Cat. No. K4500-01).
Successful cloning is demonstrated by cleavage of the plasmid pCR2.1TOPOgdhA with the restriction enzymes EcoRI and EcoRV. For this, the plasmid DNA is isolated by means of the "QIAprep Spin Plasmid Kits" (QIAGEN, Hilden, Germany) and, after cleavage, separated in a 0.8 % agarose gel.

### 5.2 Cloning of the gdhA gene in the plasmid vector pMW218

The plasmid pCR2.1TOPOgdhA is cleaved with the enzyme EcoRI, the cleavage batch is separated on 0.8% agarose gel and the gdhA fragment 2.1 kbp in size is isolated with the aid of the "QIAquick Gel Extraction Kit" (QIAGEN, Hilden, Germany). The plasmid pMW218 (Nippon Gene, Toyama, Japan) is cleaved with the enzyme EcoRI and ligated with the gdhA fragment. The E. coli strain DH5α is transformed with the ligation batch and pMW218-carrying cells are selected by plating out on LB agar (Lennox, Virology 1955, 1: 190), to which 20 µg/ml kanamycin are added.

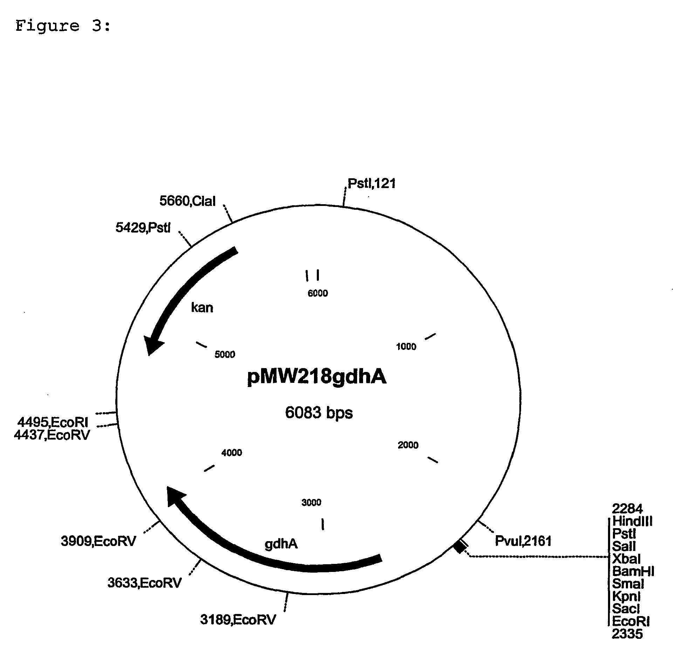

Successful cloning of the gdhA gene can be demonstrated after plasmid DNA isolation and control cleavage with EcoRI and EcoRV. The plasmid is called pMW218gdhA (Figure 3).

### 5.3 Preparation of the strain MG442ΔpckA/pMW218gdhA

The strain MG442ΔpckA obtained in Example 3 and the strain MG442 are transformed with the plasmid pMW218gdhA and transformants are selected on LB agar, which is supplemented with 20 µg/ml kanamycin. The strains MG442ΔpckA/pMW218gdhA and MG442/pMW218gdhA are formed in this manner.

### 5.4 Preparation of L-threonine

The preparation of L-threonine by the strains MG442ΔpckA/pMW218gdhA and MG442/pMW218gdhA is tested as described in Example 4. The minimal medium and the preculture medium are additionally supplemented with 20 µg/ml kanamycin.

The result of the experiment is summarized in Table 2.

**Table 2**

| Strain | OD (660 nm) | L-Threonine g/l |
|---|---|---|
| MG442 | 6.0 | 1.5 |
| MG442ΔpckA | 5.4 | 3.7 |
| MG442/pMW218gdhA | 5.6 | 2.6 |
| MG442ΔpckA/pMW218gdhA | 5.5 | 4.0 |

### Example 6

### Preparation of L-threonine with the strain MG442ΔpckA/pMW219rhtC

### 6.1 Amplification of the rhtC gene

The rhtC gene from Escherichia coli K12 is amplified using the polymerase chain reaction (PCR) and synthetic oligonucleotides. Starting from the nucleotide sequence for the rhtC gene in E. coli K12 MG1655 (gene library: Accession No. AE000458, Zakataeva et al. (FEBS Letters 452, 228-232 (1999)), PCR primers are synthesized (MWG Biotech, Ebersberg, Germany):
RhtC1 5' - CTGTTAGCATCGGCGAGGCA - 3'
RhtC2: 5' - GCATGTTGATGGCGATGACG - 3'

The chromosomal E. coli K12 MG1655 DNA employed for the PCR is isolated according to the manufacturers instructions with "QIAGEN Genomic-tips 100/G" (QIAGEN, Hilden, Germany). A DNA fragment approx. 800 bp in size can be amplified with the specific primers under standard PCR conditions (Innis et al.: PCR Protocols. A Guide to Methods and Applications, 1990, Academic Press) with Pfu-DNA polymerase (Promega Corporation, Madison, USA).

### 6.2 Cloning of the rhtC gene in the plasmid vector pMW219

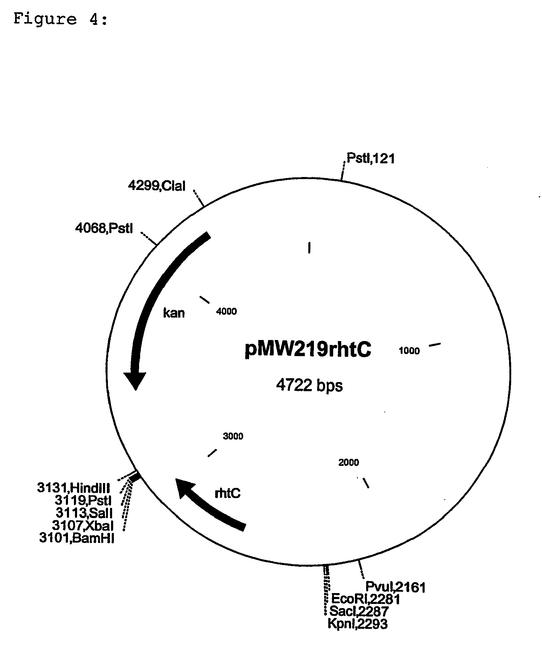

The plasmid pMW219 (Nippon Gene, Toyama, Japan) is cleaved with the enzyme SamI and ligated with the rhtC-PCR fragment. The E. coli strain DH5α is transformed with the ligation batch and pMW219-carrying cells are selected on LB agar, which is supplemented with 20 µg/ml kanamycin. Successful cloning can be demonstrated after plasmid DNA isolation and control cleavage with KpnI, HindIII and NcoI. The plasmid pMW219rhtC is shown in Figure 4.

### 6.3 Preparation of the strain MG442ΔpckA/pMW219rhtC

The strain MG442ΔpckA obtained in Example 3 and the strain MG442 are transformed with the plasmid pMW219rhtC and transformants are selected on LB agar, which is supplemented with 20 µg/ml kanamycin. The strains MG442ΔpckA/pMW219rhtC and MG442/pMW219rhtC are formed in this manner.

### 6.4 Preparation of L-threonine

The preparation of L-threonine by the strains MG442ΔpckA/pMW219rhtC and MG442/pMW219rhtC is tested as described in Example 4. The minimal medium and the preculture medium are additionally supplemented with 20 µg/ml kanamycin.

The result of the experiment is summarized in Table 3.

**Table 3**

| Strain | OD (660 nm) | L-Threonine g/l |
|---|---|---|
| MG442 | 6.0 | 1.5 |
| MG442ΔpckA | 5.4 | 3.7 |
| MG442/pMW219rhtC | 5.2 | 2.9 |
| MG442ΔpckA/pMW219rhtC | 4.8 | 4.4 |

### Example 7

### Preparation of L-threonine with the strain B-3996kurΔtdhΔpckA/pVIC40

The L-threonine-producing E. coli strain B-3996 is described in US-A- 5,175,107 and deposited at the Russian National Collection for Industrial Microorganisms (VKPM, Moscow, Russia).

The strain B-3996 has, inter alia, a resistance to α-amino-β-hydroxyvaleric acid, has an attenuated, in particular switched-off, or defective threonine dehydrogenase, has an enhanced homoserine dehydrogenase I aspartate kinase I in the feed back resistant form, has an optionally partial and compensable need for L-isoleucine and has the ability to utilize sucrose.

### 7.1 Preparation of the strain B-3996kurΔtdhΔpckA/pVIC40

After culture in antibiotic-free complete medium for approximately ten generations, a derivative of strain B-3996 which no longer contains the plasmid pVIC40 is isolated. The strain formed is streptomycin-sensitive and is designated B-3996kur.

The method described by Hamilton et al. (Journal of Bacteriology (1989) 171: 4617-4622), which is based on the use of the plasmid pMAK705 with a temperature-sensitive replicon, was used for incorporation of a deletion into the tdh gene. The plasmid pDR121 (Ravnikar and Somerville, Journal of Bacteriology (1987) 169:4716-4721) contains a DNA fragment from E. coli 3.7 kilo-base pairs (kbp) in size, on which the tdh gene is coded. To generate a deletion of the tdh gene region, pDR121 is cleaved with the restriction enzymes ClaI and EcoRV and the DNA fragment 5 kbp in size isolated is ligated, after treatment with Klenow enzyme. The ligation batch is transformed in the E. coli strain DH5α and plasmid-carrying cells are selected on LB agar, to which 50 µg/ml ampicillin are added.

Successful deletion of the tdh gene can be demonstrated after plasmid DNA isolation and control cleavage with EcoRI. The EcoRI fragment 1.7 kbp in size is isolated, and ligated with the plasmid pMAK705, which is partly digested with EcoRI. The ligation batch is transformed in DH5α and plasmid-carrying cells are selected on LB agar, to which 20 µg/ml chloramphenicol are added. Successful cloning is demonstrated after isolation of the plasmid DNA and cleavage with EcoRI. The pMAK705 derivative formed is designated pDM32.

For the gene replacement, B-3996kur is transformed with the plasmid pDM32. The replacement of the chromosomal tdh gene with the plasmid-coded deletion construct is carried out by the selection process described by Hamilton et al. and is verified by standard PCR methods (Innis et al. (1990), PCR Protocols. A Guide to Methods and Applications, Academic Press) with the following oligonucleotide primers:
Tdh1: 5'-TCGCGACCTATAAGTTTGGG-3'
Tdh2: 5'-AATACCAGCCCTTGTTCGTG-3'.

The strain formed is tested for kanamycin sensitivity and is designated B-3996kurΔtdh.

For the position-specific mutagenesis of the pckA gene, B-3996kurΔtdh is transformed with the replacement vector pMAK705ΔpckA described in Example 2. The replacement of the chromosomal pckA gene by the plasmid-coded deletion construct is carried out as described in Example 3. The strain obtained is called B-3996kurΔtdhΔpckA.

B-3996kurΔtdh and B-3996kurΔtdhΔpckA are transformed with the plasmid pVIC40 isolated from B-3996 and plasmid-carrying cells are selected on LB agar with 20 µg/ml streptomycin. In each case a selected individual colony is called B-3996kurΔtdh/pVIC40 and B-3996kurΔtdhΔpckA/pVIC40.

### 7.2 Preparation of L-threonine

The preparation of L-threonine by the strains B-3996kurΔtdh/pVIC40 and B-3996kurΔtdhΔpckA/pVIC40 is tested as described in Example 4. The minimal medium, the preculture medium and the production medium are additionally supplemented with 20 µg/ml streptomycin.

The result of the experiment is summarized in Table 4.

**Table 4**

| Strain | OD (660 nm) | L-Threonine g/l |
|---|---|---|
| B-3996kurΔtdh/pVIC40 | 4.7 | 6.26 |
| B-3996kurΔtdhΔpckA/pVIC40 | 4.9 | 8.92 |

### Example 8

### Preparation of L-lysine with the strain TOC21RΔpckA

The L-lysine-producing E. coli strain pDA1/TOC21R is described in the patent application F-A-2511032 and deposited at the Collection Nationale de Culture de Microorganisme (CNCM = National Microorganism Culture Collection, Pasteur Institute, Paris, France) under number I-167. The strain and the plasmid-free host are also described by Dauce-Le Reverend et al. (European Journal of Applied Microbiology and Biotechnology 15:227-231 (1982)) under the name TOCR21/pDA1.

### 8.1 Position-specific mutagenesis of the pckA gene in the E. coli strain TOC21R

After culture in antibiotic-free LB medium for approximately six generations, a derivative of strain pDA1/TOC21R which no longer contains the plasmid pDA1 is isolated. The strain formed is tetracycline-sensitive and is called TOC21R.

For replacement of the chromosomal pckA gene by the plasmid-coded deletion construct, TOC21R is transformed with the plasmid pMAK705ΔpckA (Example 2). The gene replacement is carried out by the selection method described by Hamilton et al. (1989) Journal of Bacteriology 174, 4617 - 4622) and is verified by standard PCR methods (Innis et al. (1990) PCR Protocols. A Guide to Methods and Applications, Academic Press) with the following oligonucleotide primers:
pckA'5'-1: 5' - GATCCGAGCCTGACAGGTTA - 3'
pckA'3'-2: 5' - CCGGAGAAGCGTAGGTGTTA - 3'

The strain obtained is called TOC21RΔpckA.

### 8.2 Preparation of L-lysine with the strain TOC21RΔpckA

The formation of L-lysine by the strains TOC21RΔ pckA and TOC21R is checked in batch cultures of 10 ml contained in 100 ml conical flasks. For this, 10 ml of preculture medium of the following composition: 2 g/l yeast extract, 10 g/l (NH₄)₂SO₄, 1 g/l KH₂PO₄, 0.5 g/l MgSO4*7H₂O, 15 g/l CaCO₃, 20 g/l glucose are inoculated and the batch is incubated for 16 hours at 37°C and 180 rpm on an ESR incubator from Kühner AG (Birsfelden, Switzerland). 250 µl of this preculture are transinoculated into 10 ml of production medium (25 g/l (NN₄)₂SO₄, 2 g/l KH₂PO₄, 1 g/l MgSO₄*7H₂O, 0.03 g/l FeSO₄*7H₂O, 0.018 g/l MnSO₄*1H₂O, 30 g/l CaCO₃ 20 g/l glucose, 25 mg/l L-isoleucine and 5 mg/l thiamine) and the batch is incubated for 72 hours at 37°C. After the incubation the optical density (OD) of the culture suspension is determined with an LP2W photometer from Dr. Lange (Berlin, Germany) at a measurement wavelength of 660 nm.

The concentration of L-lysine formed is then determined in the sterile-filtered culture supernatant with an amino acid analyzer from Eppendorf-BioTronik (Hamburg, Germany) by ion exchange chromatography and post-column reaction with ninhydrin detection.

The result of the experiment is shown in Table 5.

**Table 5**

| Strain | OD (660 nm) | L-Lysine g/l |
|---|---|---|
| TOC21R | 1.0 | 1.14 |
| TOC21RΔpckA | 1.0 | 1.27 |

### Example 9

### Preparation of L-isoleucine with the strain B-3996kurΔtdhilvA⁺ΔpckA/pVIC40

### 9.1 Preparation of the strain B-3996kurΔtdhilvA⁺ΔpckA/pVIC40

The strain B-3996kurΔtdh, which is in need of L-isoleucin, obtained in Example 7.1 is transduced with the aid of the phage P1kc (Lennox, Virology 1, 190-206 (1955); Miller, Experiments in Molecular Genetics, Cold Spring Harbor Laboratory 1972) and L-isoleucine-prototrophic transductants are isolated.

For this, the phage P1kc is multiplied on the strain MG1655 (Guyer et al., Cold Spring Harbor Symposium of Quantitative Biology 45, 135-140 (1981) and Blattner et al., Science 277, 1453-1462 (1997))and the phage lysate is employed for the transduction of the strain B-3996kurΔtdh. The multiplicity of the infection is approximately 0.2. Selection for L-isoleucine-prototrophic transductants is carried out on minimal agar, which contains 2 g/l glucose and 10 mg/l L-threonine. An L-isoleucine-prototrophic transductant is isolated, smeared on to LB agar for purification or isolation and called B-3996kurΔtdhilvA⁺.

The pckA gene of the strain B-3996kurΔtdhilvA⁺ is then replaced, as described in Example 3, by the ΔpckA allele prepared in Example 1 and 2. The strain obtained is called B-3996kurΔtdhilvA⁺ΔpckA.

The strains B-3996kurΔtdhilvA⁺ and B-3996kurΔtdhilvA⁺ΔpckA are transformed with the plasmid pVIC40 isolated from strain B-3996 and plasmid-carrying cells are selected on LB agar, which is supplemented with 20 µg/ml streptomycin. In each case a selected individual colony is called B-3996kurΔtdhilvA⁺ΔpckA/pVIC40 and B-3996kurΔtdhilvA⁺/pVIC40.

### 9.2 Preparation of L-isoleucine

The preparation of L-isoleucine by the strains B-3996kurΔtdhilvA⁺/pVIC40 and B-3996kurΔtdhilvA⁺ΔpckA/pVIC40 is tested under the test conditions as described in Example 4. The minimal medium, the preculture medium and the production medium are additionally supplemented with 20 µg/ml streptomycin.

The result of the experiment is shown in Table 6.

**Table 6**

| Strain | OD (660 nm) | L-Isoleucine mg/l |
|---|---|---|
| B-3996kurΔtdhilvA⁺/pVIC40 | 5.8 | 57 |
| B-3996kurΔtdhilvA⁺ΔpckA/pVIC40 | 5.7 | 70 |

### Example 10

### Preparation of L-valine with the strain B-12288ΔpckA

The L-valine-producing E. coli strain AJ 11502 is described in the patent specification US-A-4391907 and deposited at the National Center for Agricultural Utilization Research (Peoria, Illinois, USA) as NRRL B-12288.

### 10.1 Position-specific mutagenesis of the pckA gene in the E. coli strain B-1288

After culture in antibiotic-free LB medium for approximately six generations, a plasmid-free derivative of strain AJ 11502 is isolated. The strain formed is ampicillin-sensitive and is called AJ11502kur.

For replacement of the chromosomal pckA gene by the plasmid-coded deletion construct, AJ11502kur is transformed with the plasmid pMAK705ΔpckA (see Example 2). The gene replacement is carried out by the selection method described by Hamilton et al. (1989) Journal of Bacteriology 174, 4617 - 4622) and is verified by standard PCR methods (Innis et al. (1990) PCR Protocols. A Guide to Methods and Applications, Academic Press) with the following oligonucleotide primers:
pckA'5'-1: 5' - GATCCGAGCCTGACAGGTTA - 3'
pckA-2: 5' - CCGGAGAAGCGTAGGTGTTA - 3'

The strain obtained is called AJ11502kurΔpckA. The plasmid described in the patent specification US-A-4391907, which carries the genetic information in respect of valine production, is isolated from strain NRRL B-12288. The strain AJ11502kurΔpckA is transformed with this plasmid. One of the transformants obtained is called B-12288ΔpckA.

### 10.2 Preparation of L-valine with the strain B-12288ΔpckA

The formation of L-valine by the strains B-12288ΔpckA and NRRL B-12288 is checked in batch cultures of 10 ml contained in 100 ml conical flasks. For this, 10 ml of preculture medium of the following composition: 2 g/l yeast extract, 10 g/l (NH₄)₂SO₄, 1 g/l KH₂PO₄, 0.5 g/l MgSO₄*7H₂O, 15 g/l CaCO₃, 20 g/l glucose and 50 mg/l ampicillin are inoculated and the batch is incubated for 16 hours at 37°C and 180 rpm on an ESR incubator from Kühner AG (Birsfelden, Switzerland). 250 µl of this preculture are transinoculated into 10 ml of production medium (25 g/l (NH₄)₂SO₄, 2 g/l KH₂PO₄, 1 g/l MgSO₄*7H₂O, 0.03 g/l FeSO₄*7H₂O, 0.018 g/l MnSO₄*1H₂O, 30 g/l CaCO₃ 20 g/l glucose, 5 mg/l thiamine and 50 mg/l ampicillin) and the batch is incubated for 72 hours at 37°C. After the incubation the optical density (OD) of the culture suspension is determined with an LP2W photometer from Dr. Lange (Berlin, Germany) at a measurement wavelength of 660 nm.

The concentration of L-valine formed is then determined in the sterile-filtered culture supernatant with an amino acid analyzer from Eppendorf-BioTronik (Hamburg, Germany) by ion exchange chromatography and post-column reaction with ninhydrin detection.

The result of the experiment is shown in Table 7.

**Table 7**

| Strain | OD (660 nm) | L-Valine g/l |
|---|---|---|
| NRRL B-12288 | 5.6 | 0.93 |
| B-12288ΔpckA | 5.5 | 1.12 |

### Example 11 (no part of invention)

Construction of deletion mutations of the ytfP-yjfA gene region

The ytfP-yjfA gene region is amplified from Escherichia coli K12 using the polymerase chain reaction (PCR) and synthetic oligonucleotides. Starting from the nucleotide sequence of the ytfP-yjfA gene region in E. coli K12 MG1655 (SEQ ID No. 5), the following PCR primers are synthesized (MWG Biotech, Ebersberg, Germany):
ytfP-1: 5' - GGCGATGTCGCAACAAGCTG - 3'
ytfP-2: 5' - CTGTTCATGGCCGCTTGCTG - 3'

The chromosomal E. coli K12 MG1655 DNA employed for the PCR is isolated according to the manufacturers instructions with "Qiagen Genomic-tips 100/G" (QIAGEN, Hilden, Germany). A DNA fragment approx. 1300 bp in size can be amplified with the specific primers under standard PCR conditions (Innis et al. (1990) PCR Protocols. A Guide to Methods and Applications, Academic Press) with Taq-DNA polymerase (Gibco-BRL, Eggenstein, Germany). The PCR product is ligated with the vector pCR2.1TOPO (TOPO TA Cloning Kit, Invitrogen, Groningen, The Netherlands) in accordance with the manufacturers instructions and transformed into the E. coli strain TOP10F'. Selection of plasmid-carrying cells takes place on LB agar, to which 50 µg/ml ampicillin are added. After isolation of the plasmid DNA, successful cloning of the PCR product is checked with the restriction enzymes EcoRI and NsiI.

To generate a 337 bp deletion in the yftP-yjfA region, the vector pCR2.1TOPOytfP-yjfA is cleaved with the restriction enzymes NdeI and SspI and the DNA fragment 4.8 kbp in size is ligated, after treatment with Klenow enzyme.

To generate a 90 bp deletion, the vector pCR2.1TOPOytfPyjfA is cleaved with the enzymes NdeI and SplI and the DNA fragment 5 kbp in size is ligated, after treatment with Klenow enzyme.

The E. coli strain DH5α is transformed with the ligation batches and plasmid-carrying cells are selected on LB agar, to which 50 µg/ml ampicillin is added. After isolation of the plasmid DNA those plasmids in which the mutagenic DNA sequence shown in SEQ ID No. 6 and SEQ ID No. 7 is cloned are detected by control cleavage with the enzyme EcoRI. The plasmids are called pCR2.1TOPOΔyjfA and pCR2.1TOPOΔ90bp.

### Example 12 (no part of invention)

### Construction of the replacement vectors pMAK705ΔyjfA and pMAK705Δ90bp

The ytfP-yjfA alleles described in Example 11 are isolated from the vectors pCR2.1TOPOΔyjfA and pCR2.1TOPOΔ90bp after restriction with the enzymes SacI and XbaI and separation in 0.8% agarose gel, and ligated with the plasmid pMAK705 (Hamilton et al. (1989) Journal of Bacteriology 174, 4617 - 4622), which is digested with the enzymes SacI and XbaI.
The ligation batches are transformed in DH5α and plasmid-carrying cells are selected on LB agar, to which 20 µg/ml chloramphenicol are added. Successful cloning is demonstrated after isolation of the plasmid DNA and cleavage with the enzymes SacI and XbaI. The replacement vectors formed, pMAK705ΔyjfA (= pMAK705deltayjfA) and pMAK705Δ90bp ( = pMAK705delta90bp), are shown in Figure 2 and in Figure 5.

### Example 13 (no part of invention)

Position-specific mutagenesis of the ytfP-yjfA gene region in the E. coli strain MG442

For replacement of the chromosomal ytfP-yjfA gene region with the plasmid-coded 90 bp deletion construct, MG442 is transformed with the plasmid pMAK705Δ90bp, The gene replacement is carried out by the selection method described by Hamilton et al. (1989) Journal of Bacteriology 174, 4617 - 4622) and is verified by standard PCR methods (Innis et al. (1990) PCR Protocols. A Guide to Methods and Applications, Academic Press) with the following oligonucleotide primers:
ytfP-1: 5' - GGCGATGTCGCAACAAGCTG - 3'
ytfP-2: 5' - CTGTTCATGGCCGCTTGCTG - 3'

The strain obtained is called MG442Δ90yjfA.

### Example 14 (no part of invention)

### Preparation of L-threonine with the strain MG442Δ90yjfA

The preparation of L-threonine by the strain MG442Δ90yjfA is tested as described in Example 4. The result of the experiment is summarized in Table 8.

**Table 8**

| Strain | OD (660 nm) | L-Threonine g/l |
|---|---|---|
| MG442 | 6.0 | 1.5 |
| MG442Δ90yjfA | 5.7 | 2.1 |

### Example 15

### Preparation of L-threonine with the strain MG442Δ90yjfAΔpckA

### 15.1 Preparation of the strain MG442Δ90yjfAΔpckA

The pckA gene of the strain MG442Δ90yjfA is replaced, as described in Example 3, by the ΔpckA allele (see Example 1 and 2). The strain obtained is called MG442Δ90yjfAΔpckA.

### 15.2 Preparation of L-threonine

The preparation of L-threonine with the strain MG442Δ90yjfAΔpckA is carried out as described in Example 4. The result is shown in Table 9.

**Table 9**

| Strain | OD (660 nm) | L-Threonine g/l |
|---|---|---|
| MG442Δ90yjfA | 5.7 | 2.1 |
| MG442Δ90yjfAΔpckA | 5.3 | 3_{.}9 |

### Brief Description of the Figures:

- Figure 1: pMAK705ΔpckA ( = pMAK705deltapckA)
- Figure 2: pMAK705ΔyjfA ( = pMAK705deltayjfA)
- Figure 3: pMW218gdhA
- Figure 4: pMW219rhtC
- Figure 5: pMAK705Δ90bp ( = pMAK705delta90bp)

The length data are to be understood as approx. data. The abbreviations and designations used have the following meaning:
- cat: Chloramphenicol resistance gene
- rep-ts: Temperature-sensitive replication region of the plasmid pSC101
- pck1: Part of the 5' region of the pckA gene
- pck2: Part of the 3' region of the pckA gene
- ytfP'-yjfA': DNA sequence containing truncated coding regions of ytfP and yjfA
- kan: Kanamycin resistance gene
- gdhA: Glutamate dehydrogenase gene
- rhtC: Threonine resistance-imparting gene

The abbreviations for the restriction enzymes have the following meaning
- BamHI: restriction endonuclease from Bacillus amyloliquefaciens
- BglII: restriction endonuclease from Bacillus globigii
- ClaI: restriction endonuclease from Caryphanon latum
- EcoRI: restriction endonuclease from Escherichia coli
- EcoRV: restriction endonuclease from Escherichia coli
- HindIII: restriction endonuclease from Haemophilus influenzae
- KpnI: restriction endonuclease from Klebsiella pneumoniae
- PstI: restriction endonuclease from Providencia stuartii
- PvuI: restriction endonuclease from Proteus vulgaris
- SacI: restriction endonuclease from Streptomyces achromogenes
- SalI: restriction endonuclease from Streptomyces albus
- SmaI: restriction endonuclease from Serratia marcescens
- XbaI: restriction endonuclease from Xanthomonas badrii
- XhoI: restriction endonuclease from Xanthomonas holcicola

## Claims

1. Fermentation process for the preparation of L-amino acids, wherein the following steps are carried out:
a) fermentation of the microorganisms of the family Enterobacteriaceae producing the desired L-amino acid, in which microorganisms at least the pckA gene or nucleotide sequences coding for the pckA gene product are attenuated,
b) enrichment of the L-amino acid in the medium or in the bacterial cells, and
c) isolation of the L-amino acid, or d) constituents of the fermentation broth and the biomass in its entirety or portions thereof being isolated as a solid product together with the L-amino acid.

2. Process according to claim 1, wherein microorganisms are used in which other genes of the biosynthetic pathway of the desired L-amino acid are additionally amplified.

3. Process according to claim 1, wherein microorganisms are used in which the metabolic pathways which reduce the formation of the desired L-amino acid are at least partially switched off.

4. Process according to claim 1, wherein the expression of the polynucleotide(s) coding for the pckA gene product is switched off.

5. Process according to claim 1, wherein the regulatory and/or catalytic properties of the polypeptide (enzyme protein) coded for by the polynucleotide pckA are reduced.

6. Process according to claim 1, wherein microorganisms of the family Enterobacteriaceae are fermented, in which one or more genes selected from the group comprising:
6.1 the thrABC operon coding for aspartate kinase, homoserine dehydrogenase, homoserine kinase-and threonine synthase,
6.2 the pyc gene coding for pyruvate carboxylase,
6.3 the pps gene coding for phosphoenolpyruvate synthase,
6.4 the ppc gene coding for phosphoenolpyruvate carboxylase,
6.5 the pntA and pntB genes coding for transhydrogenase,
6.6 the rhtB gene for homoserine resistance, and
6.7 the rhtC gene for threonine resistance,
6.8 the gdhA gene coding for glutamate dehydrogenase
are simultaneously amplified.

7. Process according to claim 1, wherein microorganisms of the family Enterobacteriaceae are fermented in which one or more genes selected from the group comprising:
7.1 the tdh gene coding for threonine dehydrogenase,
7.2 the mdh gene coding for malate dehydrogenase,
7.3 the gene product of the open reading frame (orf) yjfA, and
7.4 the gene product of the open reading frame (orf) ytfP,
are attenuated or the expression is reduced.

8. Process according to claim 1, wherein L-isoleucine, L-valine, L-lysine or L-threonine is prepared.

9. Plasmid pMAK705ΔpckA containing parts of the 5' and 3' regions of the pckA gene, corresponding to SEQ ID No. 3.

10. Isolated polynucleotide from microorganisms of the family Enterobacteriaceae containing a polynucleotide sequence coding for the 5' and 3' regions of the pckA gene, shown in SEQ ID No. 4, which is particularly suitable as a constituent of plasmids for the position-specific mutagenesis of the pckA gene.

11. L-Threonine-producing strains of the family Enterobacteriaceae containing a deletion mutation in the pckA gene, corresponding to SEQ ID No. 4.

12. L-Threonine-producing strains of the family Enterobacteriaceae according to claim 11, additionally containing a deletion mutation in the open reading frame ytfP, corresponding to SEQ ID No. 6 or 7.

13. L-Threonine-producing strains of the family Enterobacteriaceae according to claim 11, additionally containing a deletion mutation in the open reading frame yjfA, corresponding to SEQ ID No. 6 or 7.

14. L-Threonine-producing strains of the family Enterobacteriaceae according to claim 11, wherein they have one or more features selected from the group comprising: a resistance to α-amino-β-hydroxyvaleric acid, an amplified homoserine dehdrogenase I-aspartate kinase I in the feed back resistant form, an optionally compensable partial need for L-isoleucine, an attenuated threonine dehydrogenase and the ability to utilize sucrose.

15. Escherichia coli K-12 strain MG442ΔpckA deposited under number DSM 13761 at the Deutsche Sammlung für Mikroorganismen und Zellkulturen (German Collection of Microorganisms and Cell Cultures).

16. Escherichia coli K-12 strain B3996kurΔtdhpckA/PVIC40, deposited under number DSM 14150 at the Deutsche Sammlung für Mikroorganismen und Zellkulturen (German Collection of Microorganisms and Cell Cultures).

## Patentansprüche

1. Verfahren zur fermentativen Herstellung von L-Aminosäuren, bei dem man folgende Schritte durchführt:
a) Fermentation der die gewünschte L-Aminosäure produzierenden Mikroorganismen der Familie Enterobacteriaceae, in denen man zumindest das pckA-Gen oder für das pckA-Genprodukt kodierende Nukleotidsequenzen abschwächt,
b) Anreicherung der L-Aminosäure im Medium oder in den Zellen der Bakterien und
c) Isolieren der L-Aminosäure oder
d) Isolieren von Bestandteilen der Fermentationsbrühe und der Biomasse in ihrer Gesamtheit oder zum Teil zusammen mit der L-Aminosäure als festes Produkt.

2. Verfahren nach Anspruch 1, bei dem man Mikroorganismen einsetzt, in denen man zusätzlich weitere Gene des Biosyntheseweges der gewünschten L-Aminosäure verstärkt.

3. Verfahren nach Anspruch 1, bei dem man Mikroorganismen einsetzt, in denen die Stoffwechselwege zumindest teilweise ausgeschaltet sind, die die Bildung der gewünschten L-Aminosäure verringern.

4. Verfahren nach Anspruch 1, bei dem man die Expression des (der) Polynukleotides (e), das (die) für das pckA-Genprodukt kodiert (kodieren) ausschaltet.

5. Verfahren nach Anspruch 1, bei dem man die regulatorischen und/oder katalytischen Eigenschaften des Polypeptids (Enzymproteins) verringert, für das das Polynukleotid pckA kodiert.

6. Verfahren nach Anspruch 1, bei dem man Mikroorganismen der Familie Enterobacteriaceae fermentiert, in denen man gleichzeitig eines oder mehrere der Gene, ausgewählt aus der Gruppe:
6.1 das für die Aspartatkinase, die Homoserin-Dehydrogenase, die Homoserinkinase und die Threoninsynthase kodierende thrABC-Operon,
6.2 das für die Pyruvat-Carboxylase kodierende pyc-Gen,
6.3 das für die Phosphoenolpyruvat-Synthase kodierende pps-Gen,
6.4 das für die Phosphoenolpyruvat-Carboxylase kodierende ppc-Gen,
6.5 die für die Transhydrogenase kodierenden Gene pntA und pntB,
6.6 das Homoserinresistenz vermittelnde Gen rhtB,
6.7 das Threoninresistenz vermittelnde Gen rhtC und
6.8 das für die Glutamat-Dehydrogenase kodierende gdhA-Gen,
verstärkt.

7. Verfahren nach Anspruch 1, bei dem man Mikroorganismen der Familie Enterobacteriaceae fermentiert, in denen man gleichzeitig eines oder mehrere der Gene, ausgewählt aus der Gruppe:
7.1 das für die Threonin-Dehydrogenase kodierende tdh-Gen,
7.2 das für die Malat-Dehydrogenase kodierende mdh-Gen,
7.3 das Genprodukt des offenen Leserahmens (orf) yjfA und
7.4 das Genprodukt des offenen Leserahmens (orf) ytfP,
abschwächt oder die Expression verringert.

8. Verfahren nach Anspruch 1, bei dem man L-Isoleucin, L-Valin, L-Lysin oder L-Threonin herstellt.

9. Plasmid pMAK705ΔpckA, das Teile der 5'- und der 3'-Region des pckA-Gens, entsprechend SEQ ID No. 3, enthält.

10. Isoliertes Polynukleotid aus Mikroorganismen der Familie Enterobacteriaceae, enthaltend eine für die 5'- und die 3'-Region des pckA-Gens kodierende Polynukleotidsequenz, dargestellt in SEQ ID No. 4, insbesondere geeignet als Bestandteil von Plasmiden für die ortsspezifische Mutagenase des pckA-Gens.

11. L-Threonin produzierende Stämme der Familie Enterobacteriaceae, enthaltend eine Deletionsmutation im pckA-Gen entsprechend SEQ ID No. 4.

12. L-Threonin produzierende Stämme der Familie Enterobacteriaceae gemäß Anspruch 11, zusätzlich enthaltend eine Deletionsmutation im offenen Leserahmen ytfP, entsprechend SEQ ID No. 6 oder 7.

13. L-Threonin produzierende Stämme der Familie Enterobacteriaceae gemäß Anspruch 11, zusätzlich enthaltend eine Deletionsmutation im offenen Leserahmen yjfA, entsprechend SEQ ID No. 6 oder 7.

14. L-Threonin produzierende Stämme der Familie Enterobacteriaceae gemäß Anspruch 11, **dadurch gekennzeichnet, dass** sie ein oder mehrere der Merkmale ausgewählt aus der Gruppe: eine Resistenz gegen α-Amino-β-hydroxyvaleriansäure, eine verstärkte Homoserin-Dehydrogenase I-Aspartatkinase I in der Feedback resistenten Form, eine gegebenenfalls kompensierbare partielle Bedürftigkeit für L-Isoleucin, eine abgeschwächte Threonin-Dehydrogenase und die Fähigkeit zur Saccharose-Verwertung besitzen.

15. Escherichia coli K-12 Stamm MG442ΔpckA, hinterlegt unter der Nummer DSM 13761 bei der Deutschen Sammlung für Mikroorganismen und Zellkulturen.

16. Escherichia coli K-12 Stamm B3996kurΔtdhpckA/pVIC40, hinterlegt unter der Nummer DSM 14150 bei der Deutschen Sammlung für Mikroorganismen und Zellkulturen.

## Revendications

1. Processus de fermentation pour la préparation d'acides aminés L, dans lequel les étapes suivantes sont effectuées :
a) une fermentation des microorganismes de la famille des Entérobactériacés produisant l'acide aminé L souhaité, microorganismes dans lesquels au moins le gène pckA ou des séquences nucléotidiques codant pour le produit du gène pckA est (sont) atténué (es) ;
b) un enrichissement de l'acide aminé L dans le milieu ou dans les cellules bactériennes ; et
c) un isolement de l'acide aminé L ou d) de constituants du bouillon de fermentation et la biomasse dans son intégralité ou bien des parties de celle-ci, étant isolés comme produit solide conjointement avec l'acide aminé L.

2. Processus selon la revendication 1, dans lequel des microorganismes sont utilisés, dans lesquels d'autres gènes de la voie de biosynthèse de l'acide aminé L souhaité sont amplifiés en plus.

3. Processus selon la revendication 1, dans lequel des microorganismes sont utilisés, dans lesquels les voies métaboliques qui réduisent la formation de l'acide aminé L souhaité sont arrêtées au moins partiellement.

4. Processus selon la revendication 1, dans lequel l'expression du (des) polynucléotide(s) codant pour le produit du gène pckA est arrêtée.

5. Processus selon la revendication 1, dans lequel les propriétés de régulation et/ou catalytiques du polypeptide (protéine enzymatique) codé par le polynucléotide pckA sont réduites.

6. Processus selon la revendication 1, dans lequel des microorganismes de la famille des Entérobactériacés sont fermentés, dans lesquels un ou plusieurs gène(s) choisi(s) dans le groupe comprenant :
6.1 l'opéron thrABC codant pour une aspartate kinase, une homosérine déshydrogénase, une homosérine kinase et une thréonine synthase,
6.2 le gène pyc codant pour une pyruvate carboxylase,
6.3 le gène pps codant pour une phosphoénolpyruvate synthase,
6.4 le gène ppc codant pour une phosphoénolpyruvate carboxylase,
6.5 les gènes pntA et pntB codant pour une transhydrogénase,
6.6 le gène rhtB pour une résistance à l'homosérine et
6.7 le gène rhtC pour une résistance à la thréonine,
6.8 le gène gdhA codant pour une glutamate déshydrogénase,
sont amplifiés simultanément.

7. Processus selon la revendication 1, dans lequel des microorganismes de la famille des Entérobactériacés sont fermentés, dans lesquels un ou plusieurs gène(s) choisi(s) dans le groupe comprenant :
7.1 le gène tdh codant pour une thréonine déshydrogénase,
7.2 le gène mdh codant pour une malate déshydrogénase,
7.3 le produit génique du cadre ouvert de lecture (orf) yjfA et
7.4 le produit génique du cadre ouvert de lecture (orf) ytfP,
sont atténués ou bien leur expression est réduite.

8. Processus selon la revendication 1, dans lequel de la L-isoleucine, de la L-valine, de la L-lysine ou de la L-thréonine est préparée.

9. Plasmide pMAK705ΔpckA contenant des parties des régions en 5' et 3' du gène pckA correspondant à la SEQ ID n°: 3.

10. Polynucléotide isolé à partir de microorganismes de la famille des Entérobactériacés contenant une séquence de polynucléotides codant pour les régions en 5' et 3' du gène pckA, présentées dans la SEQ ID n° : 4, qui est particulièrement approprié comme constituant de plasmides destinés à une mutagenèse du gène pckA spécifique d'une position.

11. Souches de la famille des Entérobactériacés productrices de L-thréonine contenant une mutation de délétion dans le gène pckA, correspondant à la SEQ ID n° : 4.

12. Souches de la famille des Entérobactériacés productrices de L-thréonine, selon la revendication 11, contenant en plus une mutation de délétion dans le cadre ouvert de lecture ytfP, correspondant à la SEQ ID n° : 6 ou à la 7.

13. Souches de la famille des Entérobactériacés productrices de L-thréonine, selon la revendication 11, contenant en plus une mutation de délétion dans le cadre ouvert de lecture yjfA, correspondant à la SEQ ID n° : 6 ou à la 7.

14. Souches de la famille des Entérobactériacés productrices de L-thréonine, selon la revendication 11, dans laquelle elles ont une ou plusieurs caractéristique(s) choisie(s) dans le groupe comprenant : une résistance à l'acide α-amino-β-hydroxyvalérique ; une homosérine déshydrogénase I-aspartate kinase I amplifiée, sous la forme résistante au rétrocontrôle ; un besoin partiel en L-isoleucine facultativement compensable ; une thréonine déshydrogénase atténuée ; et la faculté d'utiliser le saccharose.

15. Souche MG442ApckA *d'Escherichia coli* K-12 déposée sous le numéro DSM 13761 auprès du Deutsche Sammlung für Mikroorganismen und Zellkulturen (Collection allemande de microorganismes et de cultures de cellules).

16. Souche B3996kurΔtdhpckA/PVIC40 d'*Escherichia coli* K-12 déposée sous le numéro DSM 14150 auprès du Deutsche Sammlung für Mikroorganismen und Zellkulturen (Collection allemande de microorganismes et de cultures de cellules).


### SEQUENCE PROTOCOL

<110> Degussa AG
<120> Process for the fermentative preparation of L-amino acids using strains of the Enterobacteriaceae family.
<130> 000425 BT
<140>
   <141>
<160> 19
<170> PatentIn Ver. 2.1
<210> 1
   <211> 1622
   <212> DNA
   <213> Escherichia coli
<220>
   <221> CDS
   <222> (1)..(1620)
   <223> pckA
<400> 1
<210> 2
   <211> 540
   <212> PRT
   <213> Escherichia coli
<400> 2
   Met Arg Val Asn Asn Gly Leu Thr Pro Gln Glu Leu Glu Ala Tyr Gly
   1               5                   10                  15
   Ile Ser Asp Val His Asp Ile Val Tyr Asn Pro Ser Tyr Asp Leu Leu
               20                  25                  30
   Tyr Gln Glu Glu Leu Asp Pro Ser Leu Thr Gly Tyr Glu Arg Gly Val
           35                  40                  45
   Leu Thr Asn Leu Gly Ala Val Ala Val Asp Thr Gly Ile Phe Thr Gly
       50                  55                  60
   Arg Ser Pro Lys Asp Lys Tyr Ile Val Arg Asp Asp Thr Thr Arg Asp
   65                  70                  75                  80
   Thr Phe Trp Trp Ala Asp Lys Gly Lys Gly Lys Asn Asp Asn Lys Pro
                   85                  90                  95
   Leu Ser Pro Glu Thr Trp Gln His Leu Lys Gly Leu Val Thr Arg Gln
               100                 105                 110
   Leu Ser Gly Lys Arg Leu Phe Val Val Asp Ala Phe Cys Gly Ala Asn
           115                 120                 125
   Pro Asp Thr Arg Leu Ser Val Arg Phe Ile Thr Glu Val Ala Trp Gln
       130                 135                 140
   Ala His Phe Val Lys Asn Met Phe Ile Arg Pro Ser Asp Glu Glu Leu
   145                 150                 155                 160
   Ala Gly Phe Lys Pro Asp Phe Ile Val Met Asn Gly Ala Lys Cys Thr
                   165                 170                 175
   Asn Pro Gln Trp Lys Glu Gln Gly Leu Asn Ser Glu Asn Phe Val Ala
               180                 185                 190
   Phe Asn Leu Thr Glu Arg Met Gln Leu Ile Gly Gly Thr Trp Tyr Gly
           195                 200                 205
   Gly Glu Met Lys Lys Gly Met Phe Ser Met Met Asn Tyr Leu Leu Pro
       210                 215                 220
   Leu Lys Gly Ile Ala Ser Met His Cys Ser Ala Asn Val Gly Glu Lys
   225                 230                 235                 240
   Gly Asp Val Ala Val Phe Phe Gly Leu Ser Gly Thr Gly Lys Thr Thr
                   245                 250                 255
   Leu Ser Thr Asp Pro Lys Arg Arg Leu Ile Gly Asp Asp Glu His Gly
               260                 265                 270
   Trp Asp Asp Asp Gly Val Phe Asn Phe Glu Gly Gly Cys Tyr Ala Lys
           275                 280                 285
   Thr Ile Lys Leu Ser Lys Glu Ala Glu Pro Glu Ile Tyr Asn Ala Ile
       290                 295                 300
   Arg Arg Asp Ala Leu Leu Glu Asn Val Thr Val Arg Glu Asp Gly Thr
   305                 310                 315                 320
   Ile Asp Phe Asp Asp Gly Ser Lys Thr Glu Asn Thr Arg Val Ser Tyr
                   325                 330                 335
   Pro Ile Tyr His Ile Asp Asn Ile Val Lys Pro Val Ser Lys Ala Gly
               340                 345                 350
   His Ala Thr Lys Val Ile Phe Leu Thr Ala Asp Ala Phe Gly Val Leu
           355                 360                 365
   Pro Pro Val Ser Arg Leu Thr Ala Asp Gln Thr Gln Tyr His Phe Leu
       370                 375                 380
   Ser Gly Phe Thr Ala Lys Leu Ala Gly Thr Glu Arg Gly Ile Thr Glu
   385                 390                 395                 400
   Pro Thr Pro Thr Phe Ser Ala Cys Phe Gly Ala Ala Phe Leu Ser Leu
                   405                 410                 415
   His Pro Thr Gln Tyr Ala Glu Val Leu Val Lys Arg Met Gln Ala Ala
               420                 425                 430
   Gly Ala Gln Ala Tyr Leu Val Asn Thr Gly Trp Asn Gly Thr Gly Lys
           435                 440                 445
   Arg Ile Ser Ile Lys Asp Thr Arg Ala Ile Ile Asp Ala Ile Leu Asn
       450                 455                 460
   Gly Ser Leu Asp Asn Ala Glu Thr Phe Thr Leu Pro Met Phe Asn Leu
   465                 470                 475                 480
   Ala Ile Pro Thr Glu Leu Pro Gly Val Asp Thr Lys Ile Leu Asp Pro
                   485                 490                 495
   Arg Asn Thr Tyr Ala Ser Pro Glu Gln Trp Gln Glu Lys Ala Glu Thr
               500                 505                 510
   Leu Ala Lys Leu Phe Ile Asp Asn Phe Asp Lys Tyr Thr Asp Thr Pro
           515                 520                 525
   Ala Gly Ala Ala Leu Val Ala Ala Gly Pro Lys Leu
       530                 535                 540
<210> 3
   <211> 1156
   <212> DNA
   <213> Escherichia coli
<220>
   <221> misc_feature
   <222> (1)..(1156)
   <223> Mutagene DNA
<220>
   <221> misc_feature
   <222> (1)..(35)
   <223> Technical DNA/residues of the polylinker sequence
<220>
   <221> misc_feature
   <222> (36)..(522)
   <223> Part of the 5' region (pck1) of the pckA gene
<220>
   <221> misc_feature
   <222> (523)..(542)
   <223> Technical DNA/residues of the polylinker sequence
<220>
   <221> misc_feature
   <222> (543)..(1105)
   <223> Part of the 3' region (pck2) of the pckA gene
<220>
   <221> misc feature
   <222> (1106)..(1156)
   <223> Technical DNA/residues of the polylinker sequence
<400> 3
   ctagtaacgg ccgccagtgt gctggaattc ggcttgatcc gagcctgaca ggttatgagc 60
   gcggggtgtt aactaatctg ggtgccgttg ccgtcgatac cgggatcttc accggtcgtt 120
   caccaaaaga taagtatatc gtccgtgacg ataccactcg cgatactttc tggtgggcag 180
   acaaaggcaa aggtaagaac gacaacaaac ctctctctcc ggaaacctgg cagcatctga 240
   aaggcctggt gaccaggcag ctttccggca aacgtctgtt cgttgtcgac gctttctgtg 300
   gtgcgaaccc ggatactcgt ctttccgtcc gtttcatcac cgaagtggcc tggcaggcgc 360
   attttgtcaa aaacatgttt attcgcccga gcgatgaaga actggcaggt ttcaaaccag 420
   actttatcgt tatgaacggc gcgaagtgca ctaacccgca gtggaaagaa cagggtctca 480
   actccgaaaa cttcgtggcg tttaacctga ccgagcgcat gcaagccgaa ttctgcagat 540
   cctgaagatg gcactatcga ctttgatgat ggttcaaaaa ccgagaacac ccgcgtttct 600
   tatccgatct atcacatcga taacattgtt aagccggttt ccaaagcggg ccacgcgact 660
   aaggttatct tcctgactgc tgatgctttc ggcgtgttgc cgccggtttc tcgcctgact 720
   gccgatcaaa cccagtatca cttcctctct ggcttcaccg ccaaactggc cggtactgag 780
   cgtggcatca ccgaaccgac gccaaccttc tccgcttgct tcggcgcggc attcctgtcg 840
   ctgcacccga ctcagtacgc agaagtgctg gtgaaacgta tgcaggcggc gggcgcgcag 900
   gcttatctgg ttaacactgg ctggaacggc actggcaaac gtatctcgat taaagatacc 960
   cgcgccatta tcgacgccat cctcaacggt tcgctggata atgcagaaac cttcactctg 1020
   ccgatgttta acctggcgat cccaaccgaa ctgccgggcg tagacacgaa gattctcgat 1080
   ccgcgtaaca cctacgcttc tccggaagcc gaattctgca gatatccatc acactggcgg 1140
   ccgctcgagc atgcat                                                 1156
<210> 4
   <211> 1294
   <212> DNA
   <213> Escherichia coli
<220>
   <221> misc_feature
   <222> (1)..(3)
   <223> Start codon of the delta pckA allele
<220>
   <221> misc_feature
   <222> (1)..(598)
   <223> 5' region of the delta pckA allele
<220>
   <221> misc feature
   <222> (599)..(618)
   <223> Technical DNA/residues of the polylinker sequence
<220>
   <221> misc_feature
   <222> (619)..(1291)
   <223> 3' region of the delta pckA allele
<220>
   <221> misc_feature
   <222> (1292)..(1294)
   <223> Stop codon of the delta pckA allele
<400> 4
   atgcgcgtta acaatggttt gaccccgcaa gaactcgagg cttatggtat cagtgacgta 60
   catgatatcg tttacaaccc aagctacgac ctgctgtatc aggaagagct cgatccgagc 120
   ctgacaggtt atgagcgcgg ggtgttaact aatctgggtg ccgttgccgt cgataccggg 180
   atcttcaccg gtcgttcacc aaaagataag tatatcgtcc gtgacgatac cactcgcgat 240
   actttctggt gggcagacaa aggcaaaggt aagaacgaca acaaacctct ctctccggaa 300
   acctggcagc atctgaaagg cctggtgacc aggcagcttt ccggcaaacg tctgttcgtt 360
   gtcgacgctt tctgtggtgc gaacccggat actcgtcttt ccgtccgttt catcaccgaa 420
   gtggcctggc aggcgcattt tgtcaaaaac atgtttattc gcccgagcga tgaagaactg 480
   gcaggtttca aaccagactt tatcgttatg aacggcgcga agtgcactaa cccgcagtgg 540
   aaagaacagg gtctcaactc cgaaaacttc gtggcgttta acctgaccga gcgcatgcaa 600
   gccgaattct gcagatcctg aagatggcac tatcgacttt gatgatggtt caaaaaccga 660
   gaacacccgc gtttcttatc cgatctatca catcgataac attgttaagc cggtttccaa 720
   agcgggccac gcgactaagg ttatcttcct gactgctgat gctttcggcg tgttgccgcc 780
   ggtttctcgc ctgactgccg atcaaaccca gtatcacttc ctctctggct tcaccgccaa 840
   actggccggt actgagcgtg gcatcaccga accgacgcca accttctccg cttgcttcgg 900
   cgcggcattc ctgtcgctgc acccgactca gtacgcagaa gtgctggtga aacgtatgca 960
   ggcggcgggc gcgcaggctt atctggttaa cactggctgg aacggcactg gcaaacgtat 1020
   ctcgattaaa gatacccgcg ccattatcga cgccatcctc aacggttcgc tggataatgc 1080
   agaaaccttc actctgccga tgtttaacct ggcgatccca accgaactgc cgggcgtaga 1140
   cacgaagatt ctcgatccgc gtaacaccta cgcttctccg gaacagtggc aggaaaaagc 1200
   cgaaaccctg gcgaaactgt ttatcgacaa cttcgataaa tacaccgaca cccctgcggg 1260
   tgccgcgctg gtagcggctg gtccgaaact gtaa                             1294
<210> 5
   <211> 1248
   <212> DNA
   <213> Escherichia coli
<220>
   <221> gene
   <222> (376)..(714)
   <223> ORF ytfP
<220>
   <221> gene
   <222> Complement((461)..(727))
   <223> ORF yjfA
<400> 5
   ggcgatgtcg caacaagctg ccttgtctta tttgctacgt ggacaagggc tggagagcga 60
   tcagagcgac agtgcggcaa tgacctcgat gctgattggt ttgggggttg cgcaaagtgg 120
   ccagattgtg ggtaaaatcg gcgagacgtt tggcgtaagc aatttagcgc tcgacaccca 180
   gggagtaggc gactcctccc aggtagtggt cagcggctat gtattgccag gtctgcaagt 240
   gaaatacggc gtgggtatat ttgactctat agcaacactc acgttacgtt atcgcctgat 300
   gcctaagcta tatctggaag ccgtgtctgg tgtagaccag gcactggatt tgctctatca 360
   gttcgagttt tagcaatgcg aatatttgtc tacggcagtt tacgccacaa acaaggcaac 420
   agtcactgga tgaccaatgc ccagttactg ggcgatttca gtatcgataa ctaccagttg 480
   tatagcctgg gccactatcc aggcgcagtt ccggggaacg gaacggtaca cggtgaagtt 540
   tatcgtattg acaacgccac gctggccgaa cttgatgcct tgcgcaccag gggcggtgaa 600
   tacgcgcgcc agttgattca gacgccgtac gggagtgcat ggatgtacgt ttatcaacga 660
   cccgtcgatg gattaaagct aattgaaagc ggcgactggt tagacaggga taagtaacca 720
   tatgcatacg ccaccttcgg gtggcgttgt tttttgcgag acgactcgca ttctgttttg 780
   taattccctc accttttgct tttctctccg agccgctttc catatctatt aacgcataaa 840
   aaactctgct ggcattcaca aatgcgcagg ggtaaaacgt ttcctgtagc accgtgagtt 900
   atactttgta taacttaagg aggtgcagat gcgtattacc ataaaaagat gggggaacag 960
   tgcaggtatg gtcattccca atatcgtaat gaaagaactt aacttacagc cggggcagag 1020
   cgtggaggcg caagtgagca acaatcaact gattctgaca cccatctcca ggcgctactc 1080
   gcttgatgaa ctgctggcac agtgtgacat gaacgccgcg gaacttagcg agcaggatgt 1140
   ctggggtaaa tccacccctg cgggtgacga aatatggtaa agaaaagtga atttgaacgg 1200
   ggagacattg tgctggttgg ctttgatcca gcaagcggcc atgaacag              1248
<210> 6
   <211> 911
   <212> DNA
   <213> Escherichia coli
<220>
   <221> misc_feature
   <222> (1)..(911)
   <223> Deletion-carrying ytfP-yjfA region
<220>
   <221> misc_feature
   <222> (1)..(383)
   <223> 5' flank of the ytfP-yjfA region
<220>
   <221> misc_feature
   <222> (384)..(911)
   <223> 3' flank of the ytfP-yjfA region
<220>
   <221> misc_feature
   <222> (376)..(378)
   <223> ATG codon of the truncated ORF ytfP
<220>
   <221> misc_feature
   <222> Complement((388)..(390))
   <223> ATG codon of the truncated ORF yjfA
<400> 6
   ggcgatgtcg caacaagctg ccttgtctta tttgctacgt ggacaagggc tggagagcga 60
   tcagagcgac agtgcggcaa tgacctcgat gctgattggt ttgggggttg cgcaaagtgg 120
   ccagattgtg ggtaaaatcg gcgagacgtt tggcgtaagc aatttagcgc tcgacaccca 180
   gggagtaggc gactcctccc aggtagtggt cagcggctat gtattgccag gtctgcaagt 240
   gaaatacggc gtgggtatat ttgactctat agcaacactc acgttacgtt atcgcctgat 300
   gcctaagcta tatctggaag ccgtgtctgg tgtagaccag gcactggatt tgctctatca 360
   gttcgagttt tagcaatgcg aattatgcat acgccacctt cgggtggcgt tgttttttgc 420
   gagacgactc gcattctgtt ttgtaattcc ctcacctttt gcttttctct ccgagccgct 480
   ttccatatct attaacgcat aaaaaactct gctggcattc acaaatgcgc aggggtaaaa 540
   cgtttcctgt agcaccgtga gttatacttt gtataactta aggaggtgca gatgcgtatt 600
   accataaaaa gatgggggaa cagtgcaggt atggtcattc ccaatatcgt aatgaaagaa 660
   cttaacttac agccggggca gagcgtggag gcgcaagtga gcaacaatca actgattctg 720
   acacccatct ccaggcgcta ctcgcttgat gaactgctgg cacagtgtga catgaacgcc 780
   gcggaactta gcgagcagga tgtctggggt aaatccaccc ctgcgggtga cgaaatatgg 840
   taaagaaaag tgaatttgaa cggggagaca ttgtgctggt tggctttgat ccagcaagcg 900
   gccatgaaca g 911
<210> 7
   <211> 1158
   <212> DNA
   <213> Escherichia coli
<220>
   <221> misc_feature
   <222> (1)..(1158)
   <223> Deletion-carrying ytfP-yjfA region
<220>
   <221> misc_feature
   <222> (1)..(630)
   <223> 5' flank of the ytfP-yjfA region
<220>
   <221> misc feature
   <222> (631)..(11158)
   <223> 3' flank of the ytfP-yjfA region
<220>
   <221> misc_feature
   <222> (376)..(378)
   <223> ATG codon of the truncated ORF ytfP
<220>
   <221> misc feature
   <222> Complement((635)..(637))
   <223> ATG codon of the truncated ORF yjfA
<400> 7
   ggcgatgtcg caacaagctg ccttgtctta tttgctacgt ggacaagggc tggagagcga 60
   tcagagcgac agtgcggcaa tgacctcgat gctgattggt ttgggggttg cgcaaagtgg 120
   ccagattgtg ggtaaaatcg gcgagacgtt tggcgtaagc aatttagcgc tcgacaccca 180
   gggagtaggc gactcctccc aggtagtggt cagcggctat gtattgccag gtctgcaagt 240
   gaaatacggc gtgggtatat ttgactctat agcaacactc acgttacgtt atcgcctgat 300
   gcctaagcta tatctggaag ccgtgtctgg tgtagaccag gcactggatt tgctctatca 360
   gttcgagttt tagcaatgcg aatatttgtc tacggcagtt tacgccacaa acaaggcaac 420
   agtcactgga tgaccaatgc ccagttactg ggcgatttca gtatcgataa ctaccagttg 480
   tatagcctgg gccactatcc aggcgcagtt ccggggaacg gaacggtaca cggtgaagtt 540
   tatcgtattg acaacgccac gctggccgaa cttgatgcct tgcgcaccag gggcggtgaa 600
   tacgcgcgcc agttgattca gacgccgtac tatgcatacg ccaccttcgg gtggcgttgt 660
   tttttgcgag acgactcgca ttctgttttg taattccctc accttttgct tttctctccg 720
   agccgctttc catatctatt aacgcataaa aaactctgct ggcattcaca aatgcgcagg 780
   ggtaaaacgt ttcctgtagc accgtgagtt atactttgta taacttaagg aggtgcagat 840
   gcgtattacc ataaaaagat gggggaacag tgcaggtatg gtcattccca atatcgtaat 900
   gaaagaactt aacttacagc cggggcagag cgtggaggcg caagtgagca acaatcaact 960
   gattctgaca cccatctcca ggcgctactc gcttgatgaa ctgctggcac agtgtgacat 1020
   gaacgccgcg gaacttagcg agcaggatgt ctggggtaaa tccacccctg cggtgacgga 1080
   aatatggtaa agaaaagtga atttgaacgg ggagacattg tgctggttgg ctttgatcca 1140
   gcaagcggcc atgaacag                                               1158
<210> 8
   <211> 20
   <212> DNA
   <213> Artificial sequence
<220>
   <223> Description of the artificial sequence: Primer pckA'5'-1
<400> 8
   gatccgagcc tgacaggtta 20
<210> 9
   <211> 20
   <212> DNA
   <213> Artificial sequence
<220>
   <223> Description of the artificial sequence: Primer pckA'5'-2
<400> 9
   gcatgcgctc ggtcaggtta 20
<210> 10
   <211> 22
   <212> DNA
   <213> Artificial sequence
<220>
   <223> Description of the artificial sequence: Primer pckA'3'-1
<400> 10
   aggcctgaag atggcactat cg 22
<210> 11
   <211> 20
   <212> DNA
   <213> Artificial sequence
<220>
   <223> Description of the artificial sequence: Primer pckA'3'-2
<400> 11
   ccggagaagc gtaggtgtta 20
<210> 12
   <211> 20
   <212> DNA
   <213> Artificial sequence
<220>
   <223> Description of the artificial sequence: Primer Gdh1
<400> 12
   tgaacacttc tggcggtacg 20
<210> 13
   <211> 20
   <212> DNA
   <213> Artificial sequence
<220>
   <223> Description of the artificial sequence: Primer Gdh2
<400> 13
   cctcggcgaa gctaatatgg 20
<210> 14
   <211> 20
   <212> DNA
   <213> Artificial sequence
<220>
   <223> Description of the artificial sequence: Primer RhtC1
<400> 14
   ctgttagcat cggcgaggca 20
<210> 15
   <211> 20
   <212> DNA
   <213> Artificial sequence
<220>
   <223> Description of the artificial sequence: Primer RhtC2
<400> 15
   gcatgttgat ggcgatgacg 20
<210> 16
   <211> 20
   <212> DNA
   <213> Artificial sequence
<220>
   <223> Description of the artificial sequence: Primer Tdh1
<400> 16
   tcgcgaccta taagtttggg 20
<210> 17
   <211> 20
   <212> DNA
   <213> Artificial sequence
<220>
   <223> Description of the artificial sequence: Primer Tdh2
<400> 17
   aataccagcc cttgttcgtg 20
<210> 18
   <211> 20
   <212> DNA
   <213> Artificial sequence
<220>
   <223> Description of the artificial sequence: Primer ytfP-1
<400> 18
   ggcgatgtcg caacaagctg 20
<210> 19
   <211> 20
   <212> DNA
   <213> Artificial sequence
<220>
   <223> Description of the artificial sequence: Primer ytfP-2
<400> 19
   ctgttcatgg ccgcttgctg 20